# EUROPÄISCHE PATENTSCHRIFT

(11) **EP 4 159 110 B1**
(45) Veröffentlichungstag und Bekanntmachung des Hinweises auf die Patenterteilung: **24.04.2024**
(21) Anmeldenummer: 21199798.6
(22) Anmeldetag: 29.09.2021
(51) Int. Cl.: G01R 33/30, G01R 33/567, A61B 5/22, A61C 19/045, A61B 5/00, A61B 5/055

(54) **VORRICHTUNG, SYSTEM UND VERFAHREN ZUR UNTERSTÜTZUNG EINER BILDGEBUNG EINES KIEFERGELENKS**
DEVICE, SYSTEM AND METHOD FOR SUPPORTING THE IMAGING OF A TEMPOROMANDIBULAR JOINT
DISPOSITIF, SYSTÈME ET PROCÉDÉ D'AIDE À UNE IMAGERIE D'UNE ARTICULATION TEMPORO-MANDIBULAIRE

(43) Veröffentlichungstag der Anmeldung: 05.04.2023
(73) Patentinhaber: Siemens Healthineers AG, 91301 Forchheim (DE)
(72) Erfinder: Berger, Moritz, 91052 Erlangen (DE); Puls, Philipp, 91058 Erlangen (DE)
(74) Vertreter: Siemens Healthineers Patent Attorneys

(56) Entgegenhaltungen:
- US-A- 4 932 867
- US-A1- 2014 114 146
- US-A1- 2016 231 401
- Medrad: "Burnett BiDirectional TMJ Device", , 1. Januar 2001 (2001-01-01), XP055895642, Gefunden im Internet: URL:http://www.jzimaging.com/Product%20Inf o2/tmj-device.pdf [gefunden am 2022-02-25]
- Burnett1 Keith A ET AL: "Dynamic Display of the Temporomandibular Joint Meniscus by Using "Fast-Scan" MR Imaging Ad- dress reprint requests to", , 1. November 1987 (1987-11-01), XP055895524, Gefunden im Internet: URL:https://www.ajronline.org/doi/pdfplus/ 10.2214/ajr.149.5.959 [gefunden am 2022-02-25]

## Beschreibung

Die Erfindung betrifft ein Beißelement zur Unterstützung einer dynamischen Bildgebungsuntersuchung eines Kiefergelenks eines Untersuchungsobjekts. Weiterhin betrifft die Erfindung eine Bildgebungsvorrichtung mit einem erfindungsgemäßen Beißelement sowie ein Verfahren zur Durchführung einer dynamischen Bildgebungsuntersuchung eines Kiefergelenks eines Untersuchungsobjekts mit einer erfindungsgemäßen Bildgebungsvorrichtung und einem erfindungsgemäßen Beißelement.

Bei einer Bildgebung eines Kiefergelenks werden üblicherweise 2D oder 3D Bildgebungsverfahren, wie z. B. Röntgenverfahren, Computertomographieverfahren, Magnetresonanztomographieverfahren und dergleichen, eingesetzt. Die Bildgebung des Kiefergelenks erfordert dabei häufig eine sogenannte dynamische Bildgebung, bei welcher unterschiedliche Öffnungsstellungen des Kiefergelenks berücksichtigt werden, um eine Diagnose von Störungen des Kiefergelenkapparates zu ermöglichen.

Ein Erfassen von Bilddaten unterschiedlicher Öffnungsstellungen des Kiefergelenks ist jedoch insbesondere bei Verwendung von Bildgebungsverfahren mit einer erhöhten Untersuchungsdauer, wie z. B. der Magnetresonanztomographie, mit Schwierigkeiten verbunden. Durch die Bewegung des Kiefergelenks können bei erhöhten Untersuchungsdauern Bildartefakte in den erfassten Bilddaten auftreten, welche die Bildqualität negativ beeinflussen und die Diagnose erschweren. Typischerweise werden für den Zweck der Bildgebung des Kiefergelenks in unterschiedlichen Öffnungsstellungen daher Keile oder Beißstücke verwendet, welche intraoral in der Mundhöhle des Patienten positioniert werden und das Kiefergelenk in einer vorbestimmten Position arretieren. Die Öffnungsstellung des Kiefergelenks kann mittels unterschiedlich geformter Keile oder Beißstücke zwischen einzelnen Bildgebungsuntersuchungen variiert werden, bis die Bildgebung des Kiefergelenks in allen relevanten Öffnungsstellungen des Kiefergelenks erfolgt ist. Solche Verfahre sind zeitintensiv, da zwischen einzelnen Bildgebungsuntersuchungen eine Anpassung der gewünschten Öffnungsstellung des Kiefergelenks durch ein Mitglied des medizinischen Personals vorgenommen werden muss. Dabei kann ferner eine erneute Einstellung eines Bildgebungsvolumens (field of view) erforderlich sein, insbesondere wenn sich das Untersuchungsobjekt während der Einstellung der Öffnungsstellung des Kiefergelenks bewegt hat.

Aus dem Dokument Medrad: "Burnett BiDirectional TMJ Device", 1. Januar 2001 (2001-01-01), XP055895642, Gefunden im Internet: URL:http://www.jzimaging.com/Product%20Info2/tmj-device.pdf [gefunden am 2022-02-25] und der Schrift von Burnett, Keith et al.: "Dynamic Display of the Temporomandibular Joint Meniscus by Using "Fast-Scan" MR Imaging" AJR:149, November 1987, XP055895524 ist eine Vorrichtung zum präzisen Öffnen und Schließen des Kiefers während einer MR Bildgebung bekannt.

Aus der US4932867A ist eine Vorrichtung zur Bestimmung der optimalen Kieferposition unter Verwendung eines höhenverstellbaren Mittels, welches zwischen den Oberkiefer und den Unterkiefer eingesetzt wird, bekannt.

Es ist eine Aufgabe der Erfindung, eine Effizienz einer Bildgebung eines Kiefergelenks eines Untersuchungsobjekts zu erhöhen.

Diese Aufgabe wird durch die Gegenstände der unabhängigen Patentansprüche erfindungsgemäß gelöst. Vorteilhafte Ausführungsformen und zweckmäßige Weiterbildungen sind Gegenstand der Unteransprüche.

Das erfindungsgemäße Beißelement zur Unterstützung einer dynamischen Bildgebungsuntersuchung eines Kiefergelenks eines Untersuchungsobjekts umfasst einen ersten Abschnitt, einen zweiten Abschnitt, eine Kopplungseinheit und zumindest einen Sensor.

Ein Untersuchungsobjekt kann ein beliebiges Wirbeltier mit einem Kieferapparat, insbesondere ein Mensch, darstellen.

Eine Bildgebungsuntersuchung kann durch eine beliebige bildgebende Untersuchung charakterisiert sein, welche ein Erfassen von Bilddaten einer diagnostisch relevanten Körperregion eines Untersuchungsobjekts ermöglicht. Eine dynamische Bildgebungsuntersuchung stellt vorzugsweise eine Bildgebungsuntersuchung dar, bei welcher die diagnostisch relevante Körperregion, insbesondere das Kiefergelenk des Untersuchungsobjekts, während des Erfassens von Bilddaten kontinuierlich oder diskontinuierlich bewegt wird. Es ist beispielsweise vorstellbar, dass ein Patient das Kiefergelenk kontinuierlich öffnet oder schließt, während Bilddaten von dem Kiefergelenk des Patienten mittels einer Bildgebungsvorrichtung erfasst werden.

Eine Bildgebungsvorrichtung kann ein beliebiges medizinisches Gerät darstellen, welches dazu ausgebildet ist, Bilddaten der diagnostisch relevanten Körperregion des Untersuchungsobjekts zu erfassen. Vorzugsweise ist die Bildgebungsvorrichtung dazu ausgebildet, zweidimensionale, dreidimensionale, aber auch höherdimensionale, insbesondere zeitabhängige, Bilddaten des Kiefergelenks des Untersuchungsobjekts aufzunehmen. Beispiele für Bildgebungsvorrichtungen sind Magnetresonanzvorrichtungen, Röntgenvorrichtungen, Computertomographievorrichtungen, Einzelphotonen-Emissions-Computertomographievorrichtungen, Positronen-Emissions-Tomographievorrichtungen, aber auch Sonographiegeräte, elektrische Impedanztomographie und dergleichen.

In einer bevorzugten Ausführungsform ist die Bildgebungsvorrichtung als eine Magnetresonanzvorrichtung ausgestaltet. In diesem Fall kann die dynamische Bildgebungsuntersuchung ein Aussenden von hochfrequenten elektromagnetischen Wellen in eine Bildaufnahmeregion der Magnetresonanzvorrichtung und ein Erfassen von hochfrequenten Signalen aus der Bildaufnahmeregion umfassen. Die erfassten hochfrequenten Signale weisen vorzugsweise einen für Magnetresonanzuntersuchungen üblichen Frequenz- und Leistungsbereich auf.

Bei anwendungsgemäßer Positionierung des Beißelements an einer Kieferregion des Untersuchungsobjekts ist der erste Abschnitt in einer vorbestimmten relativen Position zu einem Oberkiefer des Untersuchungsobjekts positioniert und der zweite Abschnitt mechanisch mit einem Unterkiefer des Untersuchungsobjekts verbunden, wobei der erste Abschnitt und der zweite Abschnitt mechanisch mittels der Kopplungseinheit miteinander verbunden sind und in Abhängigkeit einer Bewegung des Kiefergelenks relativ zueinander positionierbar sind.

Der erste Abschnitt und/oder der zweite Abschnitt können Teilflächen oder Seiten der Kopplungseinheit darstellen. Es ist vorstellbar, dass der erste Abschnitt und der zweite Abschnitt im Wesentlichen auf gegenüberliegenden Seiten der Kopplungseinheit angeordnet sind. Insbesondere können der erste Abschnitt und/oder der zweite Abschnitt einstückig als Teile oder Teilflächen der Kopplungseinheit ausgeführt oder mechanisch mit der Kopplungseinheit verbunden sein. Eine mechanische Verbindung kann dabei eine beliebige stoffschlüssige, kraftschlüssige und/oder formschlüssige Verbindung umfassen. In einer alternativen Ausführungsform ist der erste Abschnitt dazu ausgebildet, den Oberkiefer des Untersuchungsobjekts in einer vorbestimmten relativen Position zu der Bildgebungsvorrichtung zu arretieren. Der erste Abschnitt kann hierfür ein Halteelement aufweisen, welches in einer anwendungsgemäßen Position an einem Kopf des Untersuchungsobjekts zumindest einen Abschnitt des Kopfs umgreift und/oder stützt und in der vorbestimmten relativen Position zu der Bildgebungsvorrichtung hält.

Das Beißelement kann bei anwendungsgemäßer Positionierung an der Kieferregion des Untersuchungsobjekts in eine Mundhöhle des Untersuchungsobjekts hineinragen, sodass ein wesentlicher Teil des Beißelements, z. B. mehr als ein Drittel, mehr als die Hälfte oder mehr als ein Dreiviertel des Beißelements, in der Mundhöhle des Untersuchungsobjekts positioniert ist. Es ist aber ebenso vorstellbar, dass das Beißelement bei anwendungsgemäßer Positionierung an der Kieferregion lediglich an Abschnitten des Oberkiefers und Unterkiefers, insbesondere den Frontzähnen und/oder Schneidezähnen, positioniert ist. Vorzugsweise sind der erste Abschnitt und der zweite Abschnitt derart geformt, dass sie bei anwendungsgemäßer Positionierung des Beißelements eine formschlüssige Verbindung mit den Frontzähnen des Untersuchungsobjekts ausbilden. In einer bevorzugten Ausführungsform ist der erste Abschnitt bei anwendungsgemäßer Positionierung des Beißelements auf einer dem Oberkiefer zugewandten Seite des Beißelements angeordnet, während der zweite Abschnitt auf einer dem Unterkiefer zugewandten Seite des Beißelements angeordnet ist. Es ist jedoch ebenso vorstellbar, dass der erste Abschnitt bei anwendungsgemäßer Positionierung des Beißelements an einer Wange, einer Stirn und/oder einer beliebigen anderen Kopfregion des Untersuchungsobjekts positioniert ist.

Die Kopplungseinheit kann dazu ausgebildet sein, ein Anpassen einer relativen Position des ersten Abschnitts zu dem zweiten Abschnitt zu ermöglichen. Die Kopplungseinheit ist vorzugsweise mechanisch mit dem ersten Abschnitt und dem zweiten Abschnitt verbunden. Vorzugsweise ist die Kopplungseinheit dazu ausgebildet, infolge der Bewegung des Kiefergelenks verformt zu werden. Die Kopplungseinheit kann hierfür ein Gelenk oder ein Scharnier aufweisen, welches ein Anpassen der relativen Position des ersten Abschnitts zu dem zweiten Abschnitt ermöglicht. Es ist ebenso vorstellbar, dass das Gelenk oder Scharnier ein Anpassen einer relativen Position des ersten Abschnitts und/oder des zweiten Abschnitts zu der Kopplungseinheit ermöglicht. In einer Ausführungsform ist die Kopplungseinheit dazu ausgebildet, eine Relativbewegung des ersten Abschnitts zu dem zweiten Abschnitt entlang einer Transversalebene des Untersuchungsobjekts zu begrenzen und/oder zu vermeiden. Die Kopplungseinheit kann insbesondere dazu ausgebildet sein, die Bewegung des Kiefergelenks und/oder eine Relativbewegung des Unterkiefers zu dem Oberkiefer im Wesentlichen auf eine Öffnungs- und eine Schließbewegung, insbesondere eine Bewegung entlang zwei entgegengesetzter Raumrichtungen, zu beschränken. Die zwei entgegengesetzten Raumrichtungen können dabei annähernd parallel zu einer Schnittlinie einer Transversalebene mit einer Frontalebene des Untersuchungsobjekts ausgerichtet sein.

Vorzugsweise weist die Kopplungseinheit ein Rückstellglied, wie z. B. eine Feder oder ein elastisches Element auf. Ein solches Rückstellglied kann dazu ausgebildet sein, das Beißelement, insbesondere das Gelenk oder das Scharnier, in eine Ausgangskonfiguration zu überführen und/oder der Bewegung des Kiefergelenks einen vorbestimmten Widerstand entgegenzusetzen. Es ist weiterhin vorstellbar, dass das Rückstellglied dazu ausgebildet ist, den ersten Abschnitt und den zweiten Abschnitt dem Oberkiefer und dem Unterkiefer bei einer Öffnungsbewegung des Kiefergelenks nachzuführen. Der erste Abschnitt und der zweite Abschnitt können hierbei mittels einer kraftschlüssigen Verbindung mit dem Oberkiefer und dem Unterkiefer an der Kieferregion gehalten werden. Unter den Begriffen Oberkiefer und Unterkiefer sind dabei jeweils insbesondere Zähne und/oder Zahnbögen des Oberkiefers und des Unterkiefers zu verstehen.

In einer weiteren Ausführungsform ist die Kopplungseinheit aus einem elastischen Material gefertigt, welches bei einem Schließen des Kiefers verformt wird und eine geometrische Form verändert. Ein elastisches Material kann z. B. einen natürlichen und/oder synthetischen Elastomer oder ein kompressibles Fluid umfassen. Die elastischen Rückstellkräfte des elastischen Materials können dazu geeignet sein, den ersten Abschnitt und den zweiten Abschnitt bei einer Öffnungsbewegung des Kiefergelenks dem Oberkiefer und/oder dem Unterkiefer nachführen. Insbesondere kann das Beißelement mittels der elastischen Rückstellkräfte bei einem Öffnen des Kiefergelenks in der anwendungsgemäßen Position an der Kieferregion gehalten werden. Die Kopplungseinheit kann ferner ein aktives Rückstellglied aufweisen. Beispielsweise umfasst das aktive Rückstellglied ein im Wesentlichen inkompressibles Fluid mit einem veränderlichen Volumen, welches der Öffnungsbewegung des Kiefergelenks nachführbar ist und/oder diese mittels eines vorbestimmten Fluiddrucks beeinflussen kann.

Der zumindest eine Sensor ist dazu ausgebildet, eine Information über die Bewegung des Kiefergelenks in Abhängigkeit der relativen Position des ersten Abschnitts zu dem zweiten Abschnitt zu ermitteln. Eine Information über die Bewegung des Kiefergelenks stellt vorzugsweise einen Messwert des zumindest einen Sensors dar. Der Messwert des zumindest einen Sensors kann insbesondere eine Kraftaufnahme, einen Abstand und/oder einen Druck betreffen, welcher mit der relativen Position des ersten Abschnitts zu dem zweiten Abschnitt korreliert ist. Die Information über die Bewegung des Kiefergelenks kann ferner einen Wert und/oder einen Wertebereich umfassen, welcher charakteristisch für eine Öffnungsstellung des Kiefergelenks ist. Beispielsweise kann die Information über die Bewegung des Kiefergelenks einen Winkel zwischen dem Unterkiefer und dem Oberkiefer des Untersuchungsobjekts umfassen, welcher mittels einer Recheneinheit in Abhängigkeit eines Messwerts des zumindest einen Sensors ermittelt wird. Die Information über die Bewegung des Kiefergelenks kann insbesondere zeitabhängige Werte und/oder Wertebereiche aufweisen, anhand derer sich ein zeitlicher Verlauf der Öffnungsstellung des Kiefergelenks ermitteln lässt. Es ist weiterhin vorstellbar, dass die Information über die Bewegung des Kiefergelenks eine dimensionslose oder prozentuale Quantifizierung einer Öffnungsstellung des Kiefergelenks umfasst.

In einer bevorzugten Ausführungsform weist das Beißelement eine Mehrzahl von Sensoren auf, welche dazu ausgebildet ist, die Information über die Bewegung des Kiefergelenks in Abhängigkeit der relativen Position des ersten Abschnitts zu dem zweiten Abschnitt zu ermitteln. Die Mehrzahl von Sensoren kann insbesondere Sensoren mit verschiedenen Messprinzipien umfassen.

Der zumindest eine Sensor kann ein nicht-bildgebender Sensor sein. Unter einem bildgebenden Sensor wird dabei insbesondere ein Sensor verstanden, welcher auf einer Abbildung des Untersuchungsobjekts, z. B. unter Ausnutzung eines photoelektrischen Effekts, aber auch eines Detektors oder eines Empfangssystems der Bildgebungsvorrichtung, beruht. Es ist vorstellbar, dass der zumindest eine Sensor als ein Sensor der Bildgebungsvorrichtung ausgestaltet ist. In diesem Fall ist der zumindest eine Sensor vorzugsweise als ein Detektor oder eine Antenne eines Empfangssystems der Bildgebungsvorrichtung ausgestaltet. Zum Erfassen eines Messwerts oder einer Eingangsgröße kann der zumindest eine Sensor einen messsensitiven Teil aufweisen. Der zumindest eine Sensor kann ferner einen Messwandler aufweisen, welcher einen erfassten Messwert oder eine Eingangsgröße entsprechend einer vorbestimmten Beziehung in eine Ausgangsgröße bzw. einen gewünschten Messwert umwandelt. Die Umwandlung des von dem zumindest einen Sensor erfassten Messwerts kann entsprechenden mittels des Messwandlers und/oder einer Recheneinheit des Sensors, aber auch einer Recheneinheit der Bildgebungsvorrichtung erfolgen.

Der zumindest eine Sensor kann beispielsweise einen Kraftaufnehmer, einen Positionsgeber, einen optischen Sensor, einen Füllstandsensor, einen akustischen Sensor, einen Ultraschallsensor und/oder einen Hall-Sensor aufweisen.

Bei einem Kraftaufnehmer kann der messsensitive Teil bei anwendungsgemäßer Positionierung des Beißelements derart an dem Beißelement angeordnet sein, dass der Kraftaufnehmer infolge der relativen Bewegung des ersten Abschnitts zu dem zweiten Abschnitt verformt und/oder einer Kraft ausgesetzt wird. Ein Kraftaufnehmer kann entsprechend dazu geeignet sein, ein Signal auszugeben, welches mit einer Verformung des messsensitiven Teils und/oder einer auf den messsensitiven Teil ausgeübten Kraft korreliert und/oder sich proportional dazu verhält.

Der messsensitive Teil des Kraftaufnehmers kann beispielsweise ein mechanisches Element, ein piezoelektrisches Element, ein piezoresistives Element, aber auch ein kapazitives oder ein induktives Element aufweisen. Der Kraftaufnehmer kann beispielsweise als ein Dehnungsmesstreifen ausgebildet sein, welcher eine mäanderförmige Messgitterfolie aufweist, die mechanisch mit der Kopplungseinheit verbunden und/oder in diese eingebettet ist. Ein Kraftaufnehmer kann aber auch ein Drucksensor sein, welcher dazu ausgebildet ist, eine durch ein Fluid und/oder einen Festkörper ausgeübte Kraft zu erfassen. Ein Kraftaufnehmer kann eine besonders kostengünstige und/oder einfach zu implementierende Lösung für den zumindest einen Sensor darstellen.

Ein optischer Sensor kann insbesondere als ein Abstandssensor ausgestaltet sein. Der Abstandssensor kann dazu ausgebildet sein, einen Abstand zwischen dem ersten Abschnitt und dem zweiten Abschnitt zu ermitteln. Der optische Sensor kann aber auch ein faseroptischer Sensor sein, welcher dazu ausgebildet ist, eine Verformung eines elastischen Elements der Kopplungseinheit infolge einer Transmissionsänderung von Glasfasern, welche mit dem elastischen Element verbunden sind, zu ermitteln. Ein faseroptischer Sensor kann eine Photodiode und eine Glasfaser umfassen, wobei die Glasfaser derart mit dem elastischen Element verbunden ist, dass bei einer Verformung des elastischen Elements infolge der Bewegung des Kiefergelenks auch die Glasfaser verformt wird. Vorzugsweise ist die Photodiode dazu ausgebildet, eine Intensität eines durch die Glasfaser durchgeleiteten Lichts an einer definierten Austrittsstelle der Glasfaser zu messen. Beispielsweise ist an einem Ende der Glasfaser eine lichtemittierende Diode (LED) angeordnet, welche Licht in die Glasfaser einkoppelt. An einem anderen Ende der Glasfaser ist die Photodiode angeordnet, welche an das an diesem Ende austretende Licht misst. Die mittels der Photodiode erfasste Intensität des Lichts kann als Maß für die Verformung des elastischen Elements und somit für ein Bestimmen einer relativen Bewegung des ersten Abschnitts zu dem zweiten Abschnitt herangezogen werden. Durch das Bereitstellen eines faseroptischen Sensors lässt sich ein Einsatz metallischer und/oder elektrisch leitender Komponenten des zumindest einen Sensors in einer Bildaufnahmeregion der Bildgebungsvorrichtung vermeiden. Dadurch kann eine Beeinflussung der Bildgebungsuntersuchung, insbesondere einer Magnetresonanzuntersuchung, auf vorteilhafte Weise vermieden werden.

Ein optischer Sensor, aber auch ein Ultraschallsensor, kann insbesondere als ein Abstandssensor ausgestaltet sein. Ein Abstandssensor ist beispielsweise dazu ausgebildet, die Information über die Bewegung des Kiefergelenks in Abhängigkeit des Abstands zwischen dem ersten Abschnitt und dem zweiten Abschnitt zu ermitteln. Der Abstandssensor kann insbesondere dazu ausgebildet sein, eine elektromagnetische Welle, wie z. B. ein Lichtsignal, eine Ultraschallsignal oder ein Radiofrequenz-Signal, auszusenden. Die elektromagnetische Welle kann von einer Oberfläche eines Abschnitts der Kopplungseinheit, einer Oberfläche eines Abschnitts des Oberkiefers und/oder des Unterkiefers in der Mundhöhle des Patienten und/oder eines spezifisch zum Zweck der Reflexion in den Abschnitt eingebrachten Objekts reflektiert und von dem messsensitiven Teil des Abstandssensors empfangen werden. Es ist vorstellbar, dass der Abstandssensor dazu ausgebildet ist, ein Zeitintervall zwischen dem Aussenden der elektromagnetischen Welle und dem Empfangen der reflektierten elektromagnetischen Welle zu ermitteln. Der Abstandssensor kann ebenso dazu ausgebildet sein einen Phasenversatz zwischen einer ausgesendeten und einer empfangenen elektromagnetischen Welle zu ermitteln. In Abhängigkeit des Zeitintervalls und/oder des Phasenversatzes kann der Abstandssensor ein Signal ausgeben, welches proportional zu dem Abstand zwischen dem ersten Abschnitt und dem zweiten Abschnitt ist und/oder mit diesem korreliert.

Bei Verwendung einer Magnetresonanzvorrichtung als Bildgebungsvorrichtung kann das Beißelement eine Anordnung von Hall-Sensoren aufweisen. Beispielsweise kann ein erster Hall-Sensor an dem ersten Abschnitt positioniert sein, während ein zweiter Hall-Sensor an dem zweiten Abschnitt positioniert ist. Infolge einer Bewegung des Kiefergelenks können der erste Abschnitt und der zweite Abschnitt relativ zueinander positioniert werden, wobei zumindest ein Hall-Sensor relativ zu einem statischen Grundmagnetfeld (B0-Feld) der Magnetresonanzvorrichtung ausgerichtet wird. Vorzugsweise ist der zumindest eine Hall-Sensor dazu ausgebildet, die Veränderung der Ausrichtung des zumindest einen Hall-Sensors zu dem statischen Grundmagnetfeld zu detektieren und/oder ein damit korreliertes Signal bereitzustellen. Es ist vorstellbar, dass eine Recheneinheit der Magnetresonanzvorrichtung dazu ausgebildet ist, die Information über die Bewegung des Kiefergelenks in Abhängigkeit des Signals des zumindest einen Hall-Sensors und/oder einer Abweichung eines Signals des ersten Hall-Sensors von einem Signal des zweiten Hall-Sensors zu ermitteln.

In einer alternativen Ausführungsform weist der zumindest eine Sensor eine Spule auf. Die Spule kann dazu ausgebildet sein, ein Signal bereitzustellen, welches sich infolge einer Veränderung einer Position und/oder einer Ausrichtung der Spule bei einer Bewegung des Kiefergelenks in einem Magnetfeld der Magnetresonanzvorrichtung ergibt.

Ein akustischer Sensor und/oder das Beißelement können eine Sendeeinheit umfassen, welche dazu ausgebildet ist, ein vorbestimmtes Referenzsignal, wie z. B. ein weißes Rauschen oder ein Signal mit einem vorbestimmten Frequenzspektrum, in eine Mundhöhle des Untersuchungsobjekts auszusenden. Der akustische Sensor kann weiterhin einen Schallwandler aufweisen, welcher dazu ausgebildet ist, das Referenzsignal, welches in einem Inneren der Mundhöhle reflektiert, verstärkt und/oder ausgelöscht wird, zu empfangen und in ein elektrisches Signal umzuwandeln. Der akustische Sensor und/oder eine Recheneinheit der Bildgebungsvorrichtung sind vorzugsweise dazu ausgebildet, eine Abweichung zwischen dem ausgesendeten und dem empfangenen Referenzsignal zu ermitteln und in Abhängigkeit der Abweichung die Öffnungsstellung bzw. die Bewegung des Kiefergelenks zu bestimmen. Es ist ebenso vorstellbar, dass auf ein Aussenden eines Referenzsignals mittels des akustischen Sensors verzichtet wird. In diesem Fall kann der akustische Sensor dazu ausgebildet sein, die Öffnungsstellung des Kiefergelenks in Abhängigkeit eines beliebigen Geräuschs aus einer Umgebung zu ermitteln. Ein solches Geräusch kann beispielsweise ein Geräusch aus einem Untersuchungsraum und/oder ein Summen des Untersuchungsobjekts umfassen. Der akustische Sensor kann insbesondere dazu ausgebildet sein, die Öffnungsstellung des Kiefergelenks in Abhängigkeit einer spezifischen Veränderung eines Frequenzspektrums und/oder eines Signalpegels des Geräuschs, welche infolge einer Veränderung der Resonanzeigenschaft der Mundhöhle bei einem Öffnen und Schlie-βen des Kiefergelenks auftritt, zu ermitteln. Der akustische Sensor ist bei anwendungsgemäßer Positionierung des Beißelements vorzugsweise in der Mundhöhle des Patienten positioniert.

Bei einer Kopplungseinheit mit einem im Wesentlichen inkompressiblen Fluid lässt sich ferner ein Füllstandsensor einsetzen, um die Information über die Bewegung des Kiefergelenks zu ermitteln. Beispielsweise ist das im Wesentlichen inkompressible Fluid mittels der Fluidleitung mit einem Fluidreservoir verbunden, dessen Füllstand mittels des Füllstandsensors überwacht wird. Somit lässt sich die Information über die Bewegung des Kiefergelenks in Abhängigkeit einer ermittelten Änderung des Füllstands ermitteln. Ein Füllstandsensor besitzt den Vorteil, dass der zumindest eine Sensor außerhalb eines Bildgebungsvolumens (field of view) der Bildgebungsvorrichtung positionierbar ist. Somit lässt sich ein Einfluss von Komponenten des zumindest einen Sensors auf die erfassten Bilddaten des Kiefergelenks auf vorteilhafte Weise reduzieren oder vermeiden.

Durch das Bereitstellen des erfindungsgemäßen Beißelements lässt sich die Bewegung des Kiefergelenks eines Untersuchungsobjekts während der Bildgebungsuntersuchung auf vorteilhafte Weise quantifizieren und bei einer Rekonstruktion von Bildern des Kiefergelenks berücksichtigen. Beispielsweise lassen sich die Bilder des Kiefergelenks in Abhängigkeit der Information über die Bewegung des Kiefergelenks auf vorteilhafte Weise individuellen Öffnungsstellungen des Kiefergelenks zuordnen, sodass eine Diagnose der Bewegung des Kiefergelenks vorgenommen werden kann.

In einer Ausführungsform des erfindungsgemäßen Beißelements ist der erste Abschnitt zumindest einem Abschnitt eines Zahnbogens des Oberkiefers nachgeformt. Es ist ebenso vorstellbar, dass der zweite Abschnitt zumindest einem Abschnitt eines Zahnbogens des Unterkiefers nachgeformt ist.

In einer Ausführungsform ist der zweite Abschnitt derart geformt, dass der zweite Abschnitt den Zahnbogen des Unterkiefers oder einen Teil des Zahnbogens des Unterkiefers, wie z. B. einen oder mehrere Zähne, in einer dreidimensionalen Weise umgreift. Der zweite Abschnitt kann den Zahnbogen des Unterkiefers oder den Teil des Zahnbogens des Unterkiefers dabei insbesondere an einer Innenseite des Zahnbogens (z. B. eine in Richtung der Mundhöhle weisende Seite), einer Kaufläche des Zahnbogens und/oder einer Außenseite des Zahnbogens (eine in Richtung einer Mundöffnung und/oder Wange weisende Seite) des Unterkiefers umschließen. Der zweite Abschnitt kann derart geformt sein, dass der zweite Abschnitt bei anwendungsgemäßer Positionierung des Beißelements eine formschlüssige und/oder kraftschlüssige Verbindung mit dem Zahnbogen des Unterkiefers ausbildet. Beispielsweise kann der zweite Abschnitt eine U-förmige oder V-förmige Vertiefung bzw. Aussparung aufweisen, welche dazu ausgebildet ist, einen oder mehrere Zähne des Unterkiefers aufzunehmen und/oder zu umgreifen. Es ist ebenso vorstellbar, dass der zweite Abschnitt eine dem Zahnbogen des Unterkiefers nachgeformte Gestalt aufweist und vollflächig über der Kaufläche des Zahnbogens des Unterkiefers positionierbar ist.

In einer bevorzugten Ausführungsform weist der zweite Abschnitt ein Verbindungselement auf. Das Verbindungselement kann dazu ausgebildet sein, eine kraftschlüssige und/oder formschlüssige Verbindung des zweiten Abschnitts mit dem Zahnbogen herzustellen. Beispielsweise kann das Verbindungselement als eine Klammer, eine Klemme, ein Rastelement, ein Steckelement oder dergleichen ausgeführt sein. Das Verbindungselement kann ferner als ein adhäsives Element ausgestaltet sein. Ein adhäsives Element kann dazu ausgebildet sein, eine haftende bzw. stoffschlüssige Verbindung mit dem Zahnbogen auszubilden. In einer alternativen Ausführungsform umfasst das Verbindungselement eine plastisch verformbare Masse, welche dazu ausgebildet ist, bei Kontakt mit dem Zahnbogen des Unterkiefers verformt zu werden und eine formschlüssige Verbindung mit dem Zahnbogen bereitzustellen.

In einer alternativen Ausführungsform weisen der zweite Abschnitt einen Stift oder Bolzen auf, welcher dazu ausgebildet ist, in einen Zahnzwischenraum zwischen zwei Zähnen des Untersuchungsobjekts einzugreifen. Vorzugsweise ist der Stift oder Bolzen an einer Zahninnenseite und/oder einer Zahnaußenseite mittels eines Sicherungsmechanismus des zweiten Abschnitts arretierbar, sodass der zweite Abschnitt in einer vorbestimmten relativen Position zu einem Zahnbogen des Unterkiefers fixiert ist. Ein solcher Sicherungsmechanismus kann beispielsweise eine Sicherungsmutter umfassen, welche auf ein Gewinde des Stifts oder Bolzens aufgeschraubt werden kann.

Auch der erste Abschnitt kann dem Zahnbogen des Oberkiefers nachgeformt sein, ein Verbindungselement aufweisen und/oder bei anwendungsgemäßer Positionierung des Beißelements formschlüssig, kraftschlüssig und/oder stoffschlüssig mit dem Zahnbogen des Oberkiefers verbunden sein.

Durch die erfindungsgemäße Ausgestaltung des ersten Abschnitts und/oder des zweiten Abschnitts kann das Beißelement auch bei einer Öffnungsbewegung des Kiefergelenks auf vorteilhafte Weise in der anwendungsgemäßen Position an der Kieferregion gehalten werden. Ferner lässt sich ein Komfort des Untersuchungsobjekts während der dynamischen Bildgebungsuntersuchung durch ein Bereitstellen von entsprechend geformten Abschnitten des Beißelements vorteilhaft erhöhen.

Erfindungsgemäß ist die Kopplungseinheit dazu ausgebildet, der Bewegung des Kiefergelenks unter Veränderung einer geometrischen Konfiguration einen vorbestimmten Widerstand entgegenzusetzen.

Wie oben beschrieben, kann die Kopplungseinheit ein Gelenk, ein Scharnier, ein elastisches Element oder dergleichen aufweisen. Die Kopplungseinheit kann somit dazu ausgebildet sein, eine geometrische Konfiguration der Kopplungseinheit in Abhängigkeit einer Bewegung des Kiefergelenks und einer damit verbundenen relativen Bewegung des ersten Abschnitts zu dem zweiten Abschnitt zu verändern. Eine geometrische Konfiguration kann insbesondere durch eine Form der Kopplungseinheit und/oder eine Anordnung bzw. relative Position von Komponenten der Kopplungseinheit, wie z. B. dem ersten Abschnitt, dem zweiten Abschnitt, dem Verbindungselement, aber auch dem zumindest einen Sensor, charakterisiert sein.

Wie oben beschrieben, kann die Kopplungseinheit ein elastisches Material und/oder ein Rückstellglied aufweisen, welche dazu ausgebildet sind, zumindest den zweiten Abschnitt einer Bewegung des Unterkiefers des Untersuchungsobjekts nachzuführen. Das elastische Material und/oder das Rückstellglied können der Bewegung des Kiefergelenks hierbei einen vorbestimmten Widerstand entgegensetzen. Der vorbestimmte Widerstand kann derart gewählt sein, dass eine Geschwindigkeit des Schließens des Kiefergelenks reduziert wird. Ferner können Rückstellkräfte des elastischen Materials und/oder des Rückstellglieds derart gewählt sein, dass das Kiefergelenk aus einer Okklusionsstellung ohne Unterstützung oder mit reduzierter Unterstützung des Untersuchungsobjekts in eine vorbestimmte Öffnungsstellung überführt wird. Dies kann in Abhängigkeit der Rückstellkräfte insbesondere mit einer vorbestimmten Geschwindigkeit erfolgen.

Erfindungsgemäß ist der zumindest eine Sensor dazu ausgebildet, die Information über die Bewegung des Kiefergelenks in Abhängigkeit der geometrischen Konfiguration der Kopplungseinheit zu ermitteln. Der zumindest eine Sensor kann hierfür gemäß einer oben beschriebenen Ausführungsform ausgebildet sein. Vorzugsweise ist der zumindest eine Sensor als ein Kraftaufnehmer ausgestaltet und derart an der Kopplungseinheit positioniert oder mechanisch mit der Kopplungseinheit verbunden, dass bei Änderung der geometrischen Konfiguration der Kopplungseinheit eine Kraft auf den messsensitiven Teil des Kraftaufnehmers übertragen wird.

Durch das Bereitstellen der erfindungsgemäßen Kopplungseinheit lässt sich die Geschwindigkeit der Bewegung des Kiefergelenks, insbesondere bei einer Schließbewegung des Kiefergelenks, auf vorteilhafte Weise an eine Randbedingung der Bildgebungsuntersuchung, wie z. B. eine Geschwindigkeit des Erfassens von Bilddaten, eine Echozeit, eine Repetitionszeit oder dergleichen, anpassen. Dadurch lässt sich ein Auftreten von Bildartefakten reduzieren und/oder eine Qualität erfasster Bilddaten vorteilhaft erhöhen.

In einer Ausführungsform weist die Kopplungseinheit des erfindungsgemäßen Beißelements ein Dämpfungselement auf. Das Dämpfungselement kann mit dem Rückstellglied übereinstimmen oder unabhängig von dem Rückstellglied vorliegen.

Die Kopplungseinheit ist dazu ausgebildet, eine mit einer Relativbewegung des ersten Abschnitts zu dem zweiten Abschnitt verbundene Kraft auf das Dämpfungselement zu übertragen. Die Relativbewegung des ersten Abschnitts zu dem zweiten Abschnitt kann dabei insbesondere durch eine Bewegung des Kiefergelenks verursacht sein.

Das Dämpfungselement ist dazu ausgebildet, den vorbestimmten Widerstand bereitzustellen und eine Geschwindigkeit der Bewegung des Kiefergelenks zu begrenzen. In einer bevorzugten Ausführungsform ist das Dämpfungselement als ein Kolben ausgeführt, welcher eine Kraftkopplung mit dem zweiten Abschnitt aufweist. Die Kraftkopplung kann derart ausgestaltet sein, dass eine auf den zweiten Abschnitt übertragene Kraft oder Bewegung direkt oder indirekt auf den Kolben übertragen wird. Der Kolben kann wiederum eine Kraftkopplung mit einem pneumatischen oder hydraulischen Fluid, insbesondere dem inkompressiblen Fluid, aufweisen. Es ist vorstellbar, dass das Untersuchungsobjekt bei dem Schließen des Kiefergelenks den Fluiddruck des pneumatischen oder hydraulischen Fluids überwinden muss, sodass die Bewegung des Kiefergelenks gedämpft bzw. gebremst wird. Es ist ebenso vorstellbar, dass das Untersuchungsobjekt bei dem Schließen des Kiefergelenks eine Scherviskosität oder einen viskosen Reibungswiderstand des Fluids überwinden muss. Ein Widerstand kann dabei z. B. durch eine Abmessung eines Kanals oder Spalts beeinflusst werden, durch welchen das Fluid in dem Dämpfungselement beim Öffnen und/oder Schließen des Kiefergelenks passieren muss.

Durch das Dämpfungselement lässt sich die Geschwindigkeit der Bewegung des Kiefergelenks auf vorteilhafte Weise in Abhängigkeit einer Randbedingung der Bildgebungsuntersuchung begrenzen. Ferner lässt sich mittels eines Dämpfungselements, welches eine Kraft und/oder eine Bewegung des Kiefergelenks auf ein Fluid überträgt, der zumindest eine Sensor auf vorteilhafte Weise außerhalb des Bildgebungsvolumens oder der Bildaufnahmeregion der Bildgebungsvorrichtung positionieren.

In einer bevorzugten Ausführungsform des erfindungsgemäßen Beißelements weist die Kopplungseinheit ein mit einem Fluid gefülltes Kissen auf. Ein Kissen kann einen beliebig geformten Hohlkörper aufweisen, welcher mit dem Fluid befüllt ist. Vorzugsweise ist das Kissen zumindest einem Teil eines Zahnbogens des Untersuchungsobjekts nachgeformt. Das Kissen kann eine Aussparung aufweisen, welche dazu ausgebildet ist, bei anwendungsgemäßer Positionierung des Beißelements eine Zunge des Untersuchungsobjekts aufzunehmen. Vorzugsweise weist das Kissen eine an einen Zahnbogen angepasste, insbesondere eine U-förmige oder eine dazu homöomorphe Gestalt auf.

Bei anwendungsgemäßer Positionierung des Beißelements sind der erste Abschnitt auf einer dem Oberkiefer zugewandten Seite des Kissens und der zweite Abschnitt auf einer dem Unterkiefer zugewandten Seite des Kissens angeordnet. Der erste Abschnitt und der zweite Abschnitt können bei einer Schließbewegung des Kiefergelenks insbesondere zwischen dem Oberkiefer und dem Unterkiefer zusammengedrückt und aufeinander zu bewegt werden.

Der vorbestimmte Widerstand hängt dabei von einem Fluiddruck des Fluids in dem Kissen ab. Im Falle eines kompressiblen Fluids kann das Kissen insbesondere ein geschlossenes System darstellen, welches einen Austausch des Fluids mit einer Umgebung verhindert. Das kompressible Fluid in dem Kissen kann dabei infolge der Schließbewegung des Kiefergelenks zwischen dem Oberkiefer und dem Unterkiefer komprimiert werden.

Es ist ebenso vorstellbar, dass das Fluid in dem Kissen mittels einer Fluidleitung mit einem Förderaggregat gekoppelt ist, welches dazu ausgebildet ist, den Fluiddruck in dem Kissen konstant oder dynamisch einzustellen. Das Förderaggregat kann beispielsweise als Kompressor oder Pumpe ausgestaltet sein. Das Förderaggregat ist insbesondere dazu ausgebildet, den Fluiddruck dynamisch, z. B. in Abhängigkeit einer Schließkraft und/oder einer Schließbewegung des Kiefergelenks des Untersuchungsobjekts, einzustellen.

Durch das Bereitstellen eines mit Fluid gefüllten Kissens kann ein Komfort des Untersuchungsobjekts während der Bildgebungsuntersuchung auf vorteilhafte Weise erhöht werden. Ferner lassen sich durch die Verwendung des mit Fluid gefüllten Kissens Herstellungskosten für das Beißelement auf vorteilhafte Weise reduzieren. Weiterhin kann ein mit Fluid gefülltes Kissen eine besonders geringe Interaktion mit gängigen Bildgebungsverfahren aufweisen, wodurch eine Qualität erfasster Bilddaten und/oder ein Aufwand für eine Nachbearbeitung der Bilddaten vorteilhaft reduziert werden können.

In einer weiteren Ausführungsform des erfindungsgemäßen Beißelements weist die Kopplungseinheit einen Antrieb auf, welcher dazu ausgebildet ist, den vorbestimmten Widerstand der Kopplungseinheit, die geometrische Konfiguration der Kopplungseinheit und/oder die relative Position des ersten Abschnitts zu dem zweiten Abschnitt einzustellen.

Ein Antrieb kann eine Kraftmaschine bzw. einen Motor umfassen, welcher dazu ausgebildet ist, eine Energieform, wie z. B. chemische, thermische oder elektrische Energie, in mechanische Energie, insbesondere eine gerichtete Bewegung zumindest des zweiten Abschnitts, umzuwandeln. Beispielsweise kann der Antrieb einen elektrischen Antrieb eines Förderaggregats darstellen, welches dazu ausgebildet ist, den Fluiddruck in dem Kissen einzustellen. Es ist aber ebenso vorstellbar, dass der Antrieb als ein pneumatischer oder ein hydraulischer Antrieb ausgestaltet ist. Ein solcher Antrieb kann z. B. ein Druckniveau eines vorhandenen pneumatischen oder hydraulischen Systems ausnutzen, um den Fluiddruck in dem Kissen einzustellen.

In einer weiteren Ausführungsform ist der Antrieb als ein elektrischer Antrieb oder ein Linearantrieb ausgestaltet. Beispielsweise kann zumindest der zweite Abschnitt mechanisch mit dem elektrischen Antrieb oder dem Linearabtrieb verbunden sein. Der elektrische Antrieb oder der Linearantrieb kann insbesondere dazu ausgebildet sein, den zweiten Abschnitt des Beißelements, aber auch den Unterkiefer des Untersuchungsobjekts, relativ zu dem ersten Abschnitt bzw. dem Oberkiefer des Untersuchungsobjekts zu positionieren. Dies kann bedeuten, dass der elektrische Antrieb oder der Linearantrieb dazu ausgebildet ist, eine Öffnungsbewegung des Kiefergelenks zu vollziehen oder zu unterstützen. Es ist jedoch ebenso vorstellbar, dass der elektrische Antrieb oder der Linearantrieb dazu ausgebildet ist, einer Schließbewegung des Kiefergelenks einen vorbestimmten Widerstand entgegenzusetzen. Der elektrische Antrieb oder der Linearantrieb kann ferner dazu ausgebildet sein, eine Schließbewegung des Kiefergelenks zu erkennen und in einen inaktiven Modus zu schalten. In einer alternativen Ausführungsform kann der elektrische Antrieb oder der Linearantrieb dazu ausgebildet sein, bei einer Schließbewegung des Kiefergelenks in einen Freilauf-Modus zu wechseln, um einen Widerstand zu reduzieren.

Durch das Bereitstellen eines Antriebs lässt sich der vorbestimmte Widerstand des Beißelements auf vorteilhafte Weise an individuelle Randbedingungen des Untersuchungsobjekts anpassen. Ferner lässt sich die Bewegung des Kiefergelenks auf vorteilhafte Weise unterstützen und/oder automatisieren, wodurch sich die Bewegung des Kiefergelenks verbessert an Randbedingungen der Bildgebungsuntersuchung anpassen lässt.

In einer weiteren Ausführungsform weist das erfindungsgemäße Beißelement zumindest einen Marker auf, welcher dazu ausgebildet ist, mittels der Bildgebungsuntersuchung abgebildet zu werden. Ein Marker ist vorzugsweise dazu ausgebildet, mit dem Bildgebungsverfahren der Bildgebungsvorrichtung zu interagieren. Dies kann insbesondere bedeuten, dass der Marker mittels des Bildgebungsverfahrens abbildbar ist. Beispielsweise kann der Marker dazu ausgebildet sein, eine ionisierende Strahlung zu absorbieren.

In einer bevorzugten Ausführungsform ist der zumindest eine Marker dazu ausgebildet, ein Signal zu emittieren, welches mittels einer Magnetresonanzuntersuchung abbildbar ist. Ein solcher Marker kann grundsätzlich ein aktiver und/oder ein passiver Marker sein. Ein passiver Marker lässt sich vorzugsweise mittels eines hochfrequenten Signals der Magnetresonanzvorrichtung anregen. Nach erfolgter Anregung des Markers kann der Marker selbst ein Magnetresonanzsignal aussenden, welches von einer Empfangsantenne der Magnetresonanzvorrichtung empfangen werden kann. Ein aktiver Marker ist dagegen dazu ausgebildet, ein hochfrequentes Signal auszusenden, welches direkt von der Empfangsantenne der Magnetresonanzvorrichtung empfangen werden kann. Beispiele für passive Marker sind verkapselte Fluide, wie Fischöl, Lösungen von Vitaminen und/oder andere geeignete Substanzen, welche auch andere Aggregatzustände aufweisen können. Beispiele für aktive Marker sind Spulen bzw. Antennen, welche dazu ausgebildet sind, Magnetresonanzsignale in einem Frequenz- und Leistungsbereich einer Magnetresonanzuntersuchung auszusenden.

Der zumindest eine Marker ist derart mechanisch mit dem Beißelement verbunden, dass der zumindest eine Marker bei der Bewegung des Kiefergelenks relativ zu dem ersten Abschnitt positionierbar ist. Die Magnetresonanzvorrichtung kann eine Recheneinheit aufweisen, welche dazu ausgebildet ist, in Abhängigkeit des Signals des zumindest einen Markers eine Öffnungsstellung des Kiefergelenks des Untersuchungsobjekts zu ermitteln. Der zumindest eine Marker ist vorzugsweise an dem zweiten Abschnitt des Beißelements angeordnet. Es ist ebenso vorstellbar, dass das Beißelement eine Mehrzahl von Markern aufweist, welche an unterschiedliche Positionen, insbesondere an dem ersten Abschnitt, dem zweiten Abschnitt und/oder der Kopplungseinheit, angeordnet sind. Es ist vorstellbar, dass eine erste Teilmenge der Mehrzahl von Markern derart an dem Beißelement angeordnet ist, dass die erste Teilmenge von Markern bei Bewegung des Kiefergelenks relativ zu einer zweiten Teilmenge von Markern positioniert wird. Anhand der erfassten Bilddaten kann die Bewegung des Kiefergelenks wie bei einer Skala in Abhängigkeit der relativen Positionen der ersten Teilmenge und der zweiten Teilmenge von Markern quantifiziert werden.

Mittels eines Markers kann ein Quantifizieren der Bewegung des Kiefergelenks auf vorteilhafte Weise unterstützt werden. Dadurch lässt sich ein Zeitaufwand und/oder eine Genauigkeit des Quantifizierens der Bewegung des Kiefergelenks auf vorteilhafte Weise erhöhen.

Die erfindungsgemäße Bildgebungsvorrichtung ist dazu ausgebildet, eine dynamische Bildgebungsuntersuchung eines Kiefergelenks eines Untersuchungsobjekts während einer Bewegung des Kiefergelenks durchzuführen.

Die Bildgebungsvorrichtung kann gemäß einer oben beschriebenen Ausführungsform ausgestaltet sein. Vorzugsweise ist die Bildgebungsvorrichtung als eine Magnetresonanzvorrichtung ausgestaltet. Die Magnetresonanzvorrichtung kann eine Halterung umfassen, welche dazu ausgebildet ist, das Beißelement in der anwendungsgemäßen Position an der Kieferregion des Untersuchungsobjekts zu halten. Die Halterung kann mechanisch mit der Magnetresonanzvorrichtung, insbesondere einer Patientenlagerungsvorrichtung und/oder einem Patiententisch, verbunden sein. Es ist aber ebenso vorstellbar, dass die Halterung eine von der Magnetresonanzvorrichtung getrennte Komponente ist. Die Halterung kann in diesem Fall an einer Wand und/oder einer Decke eines Untersuchungsraums der Magnetresonanzvorrichtung montiert sein oder reversibel an der Patientenlagerungsvorrichtung angebracht sein. Vorzugsweise weist die Halterung eine Positionierungseinheit auf, welche dazu ausgebildet ist, eine Position der Halterung und/oder der Lokalspule relativ zu der Patientenlagerungsvorrichtung und/oder dem Untersuchungsobjekt einzustellen. Es ist ferner vorstellbar, dass die Positionierungseinheit dazu ausgelegt ist, eine räumliche Position und/oder Orientierung des Beißelements und/oder der Halterung einzustellen.

Die Magnetresonanzvorrichtung ist vorzugsweise dazu ausgebildet, Bilddaten des Kiefergelenks des Untersuchungsobjekts zu erfassen und Bilder des Kiefergelenks in Abhängigkeit der erfassten Bilddaten zu rekonstruieren. Hierfür kann die Magnetresonanzvorrichtung insbesondere eine Recheneinheit aufweisen, welche dazu konfiguriert ist, die Bilder des Kiefergelenks in Abhängigkeit der erfassten Bilddaten zu rekonstruieren. Die Bildgebungsvorrichtung kann hierfür selbstverständlich weitere Komponenten umfassen, welche für eine ordnungsgemäße Funktion der Bildgebungsvorrichtung erforderlich sind.

Durch die erfindungsgemäße Bildgebungsvorrichtung kann auf vorteilhafte Weise eine zeiteffiziente und reproduzierbare Aufnahme von Bilddaten des Kiefergelenks des Untersuchungsobjekts ermöglicht werden. Die erfindungsgemäße Bildgebungsvorrichtung teilt die Vorteile des erfindungsgemäßen Beißelements gemäß einer oben beschriebenen Ausführungsform.

Bei dem erfindungsgemäßen Verfahren zur Durchführung einer dynamischen Bildgebungsuntersuchung eines Kiefergelenks eines Untersuchungsobjekts mit einer Bildgebungsvorrichtung gemäß einer oben beschriebenen Ausführungsform und einem Beißelement gemäß einer oben beschriebenen Ausführungsform ist das Beißelement in einer anwendungsgemäßen Position an der Kieferregion des Untersuchungsobjekts positioniert. Das erfindungsgemäße Verfahren umfasst die folgenden Schritte:
- Erfassen von Bilddaten des Kiefergelenks des Untersuchungsobjekts bei verschiedenen relativen Positionen des ersten Abschnitts zu dem zweiten Abschnitt des Beißelements mittels der Bildgebungsvorrichtung,
- Erfassen der Information über die Bewegung des Kiefergelenks mittels des zumindest einen Sensors und
- Rekonstruieren von Bildern des Kiefergelenks des Untersuchungsobjekts in Abhängigkeit der Information bezüglich der Bewegung des Kiefergelenks.

Vor der dynamischen Bildgebungsuntersuchung wird das Untersuchungsobjekt vorzugsweise für die Bildgebungsuntersuchung vorbereitet und in einer vorbestimmten relativen Position zu der Bildgebungsvorrichtung positioniert. In entsprechender Weise kann das Beißelement, z. B. mittels einer Positionierungseinheit und/oder einer Halterung, in der anwendungsgemä-βen Position an der Kieferregion des Untersuchungsobjekts positioniert werden.

Das Erfassen der Bilddaten des Kiefergelenks des Untersuchungsobjekts erfolgt bei verschiedenen relativen Positionen des ersten Abschnitts zu dem zweiten Abschnitt des Beißelements. Insbesondere kann das Erfassen der Bilddaten des Kiefergelenks während eines Variierens der relativen Position des ersten Abschnitts zu dem zweiten Abschnitt des Beißelements erfolgen. Die relative Position des ersten Abschnitts zu dem zweiten Abschnitt kann dabei kontinuierlich (z. B. stetig), aber auch diskontinuierlich (z. B. in diskreten Zeitschritten), variiert werden. In einer Ausführungsform erfolgt das Variieren der relativen Position des ersten Abschnitts zu dem zweiten Abschnitt infolge der Bewegung, insbesondere eines wiederholenden Öffnens und Schließens, des Kiefergelenks durch das Untersuchungsobjekt. Ein vorbestimmter Widerstand des Beißelements kann dabei eine Ausführung und/oder eine Geschwindigkeit der Bewegung des Kiefergelenks limitieren oder beeinflussen. Es ist aber ebenso vorstellbar, dass das Beißelement dazu ausgebildet ist, die Bewegung des Kiefergelenks zu unterstützen und/oder vorzugeben. Eine Recheneinheit und/oder eine Steuereinheit der Bildgebungsvorrichtung kann hierfür einen Antrieb gemäß einer oben beschriebenen Ausführungsform ansteuern. Durch das Variieren der relativen Position des ersten Abschnitts zu dem zweiten Abschnitt des Beißelements während der Bildgebungsuntersuchung lassen sich Bilddaten des Kiefergelenks bei verschiedenen Öffnungsstellungen des Kiefergelenks erfassen. In einer bevorzugten Ausführungsform umfasst das Erfassen von Bilddaten des Kiefergelenks ein Erfassen von räumlich kodierten Magnetresonanzsignalen und/oder k-Raum Daten mittels einer Magnetresonanzvorrichtung.

Das Erfassen der Information über die Bewegung des Kiefergelenks mittels des zumindest einen Sensors kann beispielsweise ein Erfassen eines Abstands zwischen dem ersten Abschnitt und dem zweiten Abschnitt mittels eines Positionsgebers und/oder eines Abstandssensors, insbesondere eines akustischen Sensors, eines Ultraschallsensors, eines LASER-Abstandssensors, eines induktiven Sensors und/oder eines kapazitiven Sensors, umfassen. Es ist aber ebenso vorstellbar, dass das Erfassen der Information über die Bewegung des Kiefergelenks mittels eines Kraftaufnehmers erfolgt, welcher beispielsweise eine von dem Unterkiefer des Untersuchungsobjekts auf den zweiten Abschnitt ausgeübte Kraft ermittelt. Dabei kann die Recheneinheit der Bildgebungsvorrichtung die Bewegung des Kiefergelenks in Abhängigkeit der ermittelten Kraft bestimmen. Ferner kann die Recheneinheit die Information über die Bewegung des Kiefergelenks in Abhängigkeit eines Füllstands bzw. eines Füllstandsensors gemäß einer oben beschriebenen Ausführungsform ermitteln. In einer bevorzugten Ausführungsform des erfindungsgemäßen Verfahrens finden die Schritte des Erfassens von Bilddaten des Kiefergelenks und des Erfassens der Information über die Bewegung des Kiefergelenks mittels des zumindest einen Sensors zeitgleich statt. Es ist ebenso vorstellbar, dass sich die beiden Schritte zumindest teilweise zeitlich überlagern. Das Erfassen der Bilddaten des Kiefergelenks erfolgt vorzugsweise in Abhängigkeit der erfassten Information über die Bewegung des Kiefergelenks. Insbesondere kann das Erfassen der Bilddaten des Kiefergelenks in Abhängigkeit der Information über die Bewegung des Kiefergelenks mittels eines Gating-Verfahrens erfolgen.

Das erfindungsgemäße Verfahren kann insbesondere den Schritt eines Zuordnens einer relativen Position des ersten Abschnitts zu dem zweiten Abschnitt zu den erfassten Bilddaten in Abhängigkeit der Information über die Bewegung des Kiefergelenks umfassen.

Beispielsweise können die erfassten Bilddaten anhand der Information über die Bewegung des Kiefergelenks ausgewählten Öffnungsstellungen des Kiefergelenks zugeordnet werden. Es ist ebenso vorstellbar, dass erfasste Bilddaten des Kiefergelenks einem Wert und/oder einem Wertebereich eines Messwerts des zumindest einen Sensors zugeordnet werden, welche für eine bestimmte Öffnungsstellung des Kiefergelenks charakteristisch sind. Dadurch lassen sich die erfassten Bilddaten einer Öffnungsstellung oder einer Mehrzahl von Öffnungsstellungen des Kiefergelenks zuordnen. Es ist aber ebenso vorstellbar, dass in Abhängigkeit der zeitabhängigen Messwerte des zumindest einen Sensors eine zeitabhängige Bewegung bzw. eine Bewegungshistorie des Kiefergelenks ermittelt wird. Erfasste Bilddaten des Kiefergelenks können dabei anhand eines Zeitstempels einer Öffnungsstellung des Kiefergelenks zugeordnet werden.

Vorzugsweise werden bei dem Rekonstruieren von Bildern des Kiefergelenks bestimmten Öffnungsstellungen des Kiefergelenks zugeordnete Bilder in Abhängigkeit der erfassten Bilddaten erstellt. Das Rekonstruieren von Bildern des Kiefergelenks des Untersuchungsobjekts in Abhängigkeit der Information über die Bewegung des Kiefergelenks kann aber auch ein Zuordnen von Bildern des Kiefergelenks zu bestimmten Öffnungsstellungen des Kiefergelenks umfassen. Eine Anzahl von Öffnungsstellungen des Kiefergelenks, von denen Bilder rekonstruiert werden, kann dabei durch eine Geschwindigkeit der Bewegung des Kiefergelenks bestimmt sein und/oder durch eine Randbedingung der Bildgebungsuntersuchung, aber auch den vorbestimmten Widerstand des Beißelements, vorgegeben oder beeinflusst werden. In einer möglichen Ausführungsform des erfindungsgemäßen Verfahrens werden einzelne Bilder des Kiefergelenks in Abhängigkeit eines mittels des zumindest einen Sensors erfassten Messwerts einzelnen Öffnungswinkeln des Kiefergelenks zugeordnet.

In einer Ausführungsform umfasst das erfindungsgemäße Verfahren ein Erstellen eines Modells eines Oberkiefers und/oder eines Unterkiefers des Untersuchungsobjekts, wobei das Rekonstruieren der Bilder des Kiefergelenks in Abhängigkeit des Modells des Oberkiefers und/oder des Unterkiefers erfolgt. Es ist insbesondere vorstellbar, dass das Zuordnen der Bilder des Kiefergelenks zu Öffnungsstellungen des Kiefergelenks in Abhängigkeit des Modells des Oberkiefers und/oder Unterkiefers erfolgt. Durch das Verwenden eines Modells lassen sich beliebige Öffnungsstellungen des Kiefergelenks abbilden und/oder eine beliebig zeitlich aufgelöste Repräsentation eines Bewegungsvorgangs des Kiefergelenks bereitstellen. Diese Informationen lassen sich vorteilhaft bei dem Zuordnen einzelner Bilder zu einzelnen Öffnungsstellungen des Kiefergelenks verwenden, um eine hohe Qualität von Bildern des Kiefergelenks zu erreichen und/oder ein Auftreten von Bildartefakten zu reduzieren oder zu vermeiden.

Vorzugsweise werden in einem weiteren Schritt des erfindungsgemäßen Verfahrens die mittels der Recheneinheit der Bildgebungsvorrichtung rekonstruierten Bilder des Kiefergelenks des Untersuchungsobjekts an eine Ausgabeeinheit und/oder eine Speichereinheit bereitgestellt.

Mittels des erfindungsgemäßen Verfahrens lässt sich eine besonders einfache und/oder effiziente Möglichkeit für eine dynamische Bildgebungsuntersuchung des Kiefergelenks des Untersuchungsobjekts bereitstellen. Ferner kann das erfindungsgemäße Verfahren auf vorteilhafte Weise auch bei Bildgebungsvorrichtungen mit relativ langen Untersuchungsdauern eingesetzt werden, da ein Auftreten von Bildartefakten durch das Rekonstruieren der Bilder des Kiefergelenks in Abhängigkeit der Information über die Bewegung des Kiefergelenks reduziert wird. Das erfindungsgemäße Verfahren teilt die Vorteile der erfindungsgemäßen Bildgebungsvorrichtung.

In einer Ausführungsform des erfindungsgemäßen Verfahrens umfasst das Rekonstruieren der Bilder des Kiefergelenks ein Ermitteln einer Mehrzahl von Öffnungsstellungen des Kiefergelenks in Abhängigkeit der Information über die Bewegung des Kiefergelenks, wobei die Bilder des Kiefergelenks der Mehrzahl von Öffnungsstellungen des Kiefergelenks zugeordnet werden.

Das Ermitteln einer Öffnungsstellung des Kiefergelenks kann gemäß einer oben beschriebenen Ausführungsform erfolgen. Vorzugsweise erfolgt das Ermitteln einer Öffnungsstellung in Abhängigkeit eines Signals oder Messwerts des zumindest einen Sensors, wie z. B. einer Kraftaufnahme eines Abschnitts des Beißelements bzw. der Kopplungseinheit, eines Füllstands eines Fluidreservoirs, eines Fluiddrucks eines Fluids oder eines Abstands zwischen dem ersten Abschnitt und dem zweiten Abschnitt. Die Information über die Bewegung des Kiefergelenks umfasst vorzugsweise zeitabhängige Messwerte des zumindest einen Sensors, wie z. B. Messwerte mit korrespondierenden Zeitstempeln oder eine Messwert-Zeit-Kurve.

Es ist vorstellbar, dass ein oder mehrere Bilder der Kieferregion einem Wert und/oder einem Wertebereich eines Messwerts des zumindest einen Sensors zugeordnet werden, welche für eine bestimmte Öffnungsstellung charakteristisch sind. Dadurch lassen sich die Bilder des Kiefergelenks einer Öffnungsstellung oder einer Mehrzahl von Öffnungsstellungen des Kiefergelenks zuordnen. Es ist aber ebenso vorstellbar, dass in Abhängigkeit der zeitabhängigen Messwerte des zumindest einen Sensors eine zeitabhängige Bewegung bzw. eine Bewegungshistorie des Kiefergelenks ermittelt wird. Einzelne Bilder des Kiefergelenks können dabei anhand eines Zeitstempels einer Öffnungsstellung des Kiefergelenks zugeordnet werden. Hierbei können insbesondere auch Verfahren der Interpolation oder der Ausgleichsrechnung auf die zeitabhängigen Messwerte bzw. die Messwert-Zeit-Kurve angewandt werden. Das Zuordnen der Bilder des Kiefergelenks zu der Mehrzahl von Öffnungsstellungen des Kiefergelenks kann weiterhin auch in Abhängigkeit eines Modells des Oberkiefers und/oder Unterkiefers erfolgen.

Mittels des Zuordnens von Bildern des Kiefergelenks zu einer Mehrzahl von Öffnungsstellungen des Kiefergelenks lässt sich das Verfahren auf vorteilhafte Weise an unterschiedliche Geschwindigkeiten der Kieferbewegung des Untersuchungsobjekts anpassen. Ferner lassen sich eine Auflösung eines Bewegungsvorgangs des Kiefergelenks, aber auch eine Qualität der Bilder des Kiefergelenks, auf vorteilhafte Weise in Abhängigkeit einer Dauer bzw. einer Anzahl von Öffnungs- und Schließvorgängen des Kiefergelenks anpassen.

In einer Ausführungsform des erfindungsgemäßen Verfahrens umfasst das Erfassen der Information über die Bewegung des Kiefergelenks ein Erfassen von Bilddaten des zumindest einen Markers, wobei das Rekonstruieren der Bilder des Kiefergelenks ein Ermitteln der Mehrzahl von Öffnungsstellungen des Kiefergelenks in Abhängigkeit der erfassten Bilddaten des zumindest einen Markers umfasst.

Der Schritt des Erfassens der Information über die Bewegung des Kiefergelenks überlagert sich vorzugsweise zumindest teilweise mit dem Schritt des Erfassens von Bilddaten des Kiefergelenks. Es ist vorstellbar, dass das Ermitteln einer Öffnungsstellung des Kiefergelenks in Abhängigkeit einer Position des Markers, insbesondere einer relativen Position des Markers zu einer Kieferstruktur und/oder einem weiteren Marker, erfolgt. Eine Kieferstruktur kann dabei eine anatomische Struktur, insbesondere eine Landmarke, darstellen, welche mittels der Recheneinheit in Abhängigkeit der erfassten Bilddaten und/oder Bilder der Kieferregion identifiziert und einer räumlichen Position oder einer relativen Position zu dem Marker zugeordnet werden kann. In einem Beispiel weist das Beißelement einen ersten Marker und einen zweiten Marker auf, wobei der erste Marker an dem ersten Abschnitt positioniert ist, während der zweite Marker an dem zweiten Abschnitt positioniert ist. Durch das Bewegen des Kiefergelenks werden der erste Abschnitt und der zweite Abschnitt relativ zueinander positioniert. Dadurch können auch der erste Marker und der zweite Marker relativ zueinander positioniert werden, wobei die Öffnungsstellung des Kiefergelenks mit der relativen Position des ersten Abschnitts und des zweiten Abschnitts korreliert ist. Vorzugsweise weist die Recheneinheit eine Bildverarbeitungseinheit auf, welche Mehrzahl von Öffnungsstellungen des Kiefergelenks in Abhängigkeit von relativen Positionen des ersten Abschnitts zu dem zweiten Abschnitt ermittelt. Die erfassten Bilddaten können dabei mittels einer regulären Bildgebungssequenz, aber auch einer Scout- oder Navigatormessung, erfasst werden.

Durch das Verwenden eines Markers lässt sich das Ermitteln verschiedener Öffnungsstellungen des Kiefergelenks auf vorteilhafte Weise in Abhängigkeit von bereits erfassten Bilddaten und/oder Bildern der Kieferregion durchführen. Dadurch kann ein Rechenaufwand des erfindungsgemäßen Verfahrens reduziert werden. Ferner lässt sich durch ein Überwachen oder Tracken eines ersten Markers und eines zweiten Markers, aber auch einer Landmarke, die geometrische Konfiguration des Beißelements ermitteln. In Abhängigkeit der geometrischen Konfiguration des Beißelements kann eine dazugehörige Öffnungsstellung des Kiefergelenks vorteilhaft mit besonders hoher Genauigkeit bestimmt werden.

Gemäß einer weiteren Ausführungsform des erfindungsgemäßen Verfahrens umfasst das Erfassen von Bilddaten des Kiefergelenks und/oder das Rekonstruieren von Bildern des Kiefergelenks einen Einsatz eines Motion-Gating-Verfahrens.

Vorzugsweise erfolgt das Erfassen von Bilddaten des Kiefergelenks in Abhängigkeit eines Signals des zumindest einen Sensors bzw. der Information über die Bewegung des Kiefergelenks. Dies kann bedeuten, dass ein Erfassen von Bilddaten des Kiefergelenks genau dann erfolgt, wenn das Signal des zumindest einen Sensors und/oder die Information über die Bewegung des Kiefergelenks einen vorbestimmten Wert und/oder Wertebereich aufweist. Das Signal des zumindest einen Sensors kann dabei einer relativen Position des ersten Abschnitts zu dem zweiten Abschnitt, insbesondere einer Öffnungsstellung des Kiefergelenks, zugeordnet sein. Somit lässt sich das Erfassen von Bilddaten auf eine oder mehrere vorbestimmte Öffnungsstellungen beschränken.

Es ist jedoch ebenso vorstellbar, dass ein Auswählen bereits erfasster Bilddaten des Kiefergelenks, welche für das Rekonstruieren der Bilder des Kiefergelenks verwendet werden, in Abhängigkeit eines Signals des zumindest einen Sensors bzw. der Information über die Bewegung des Kiefergelenks erfolgt. Hierbei können erfasste Bilddaten nur dann für das Rekonstruieren von Bildern des Kiefergelenks herangezogen werden, wenn das Signal des zumindest einen Sensors und/oder die Information über die Bewegung des Kiefergelenks einen vorbestimmten Wert und/oder einen vorbestimmten Wertebereich aufweisen.

In einer Ausführungsform werden erfasste Bilddaten des Kiefergelenks genau dann für das Rekonstruieren von Bildern des Kiefergelenks verwendet, wenn ein Abstand zwischen dem ersten Abschnitt und dem zweiten Abschnitt einen bestimmten Wert beträgt oder in einem vorbestimmten Wertebereich liegt. Gleichermaßen können erfasste Bilddaten des Kiefergelenks für das Rekonstruieren von Bildern des Kiefergelenks einer vorbestimmten Öffnungsstellung des Kiefergelenks genau dann verwendet werden, wenn eine Kraftaufnahme, ein Füllstand, ein Fluiddruck und/oder eine Position eines Markers einen vorbestimmten Wert aufweisen und/oder in einem vorbestimmten Wertebereich liegen. Dabei können einzelnen Öffnungsstellungen des Kiefergelenks einzelne Werte oder Wertebereiche des Signals des zumindest einen Sensors und/oder der Information über die Bewegung des Kiefergelenks zugeordnet sein. Es ist vorstellbar, dass sich auch bereits rekonstruierte Bilder des Kiefergelenks auf diese Weise einzelnen Öffnungsstellungen des Kiefergelenks zuordnen lassen. Weiterhin können Bilder oder Bilddaten des Kiefergelenks, welche einer Öffnungsstellung des Kiefergelenks zugeordnet sind, insbesondere auch überlagert werden.

In einer weiteren Ausführungsform kann das Motion-Gating-Verfahren auch ein Aussortieren erfasster Bilddaten und/oder Bilder des Kiefergelenks umfassen, wenn die erfassten Bilddaten und/oder Bilder des Kiefergelenks keiner vorbestimmten und/oder gewünschten Öffnungsstellung des Kiefergelenks zugeordnet sind. Es ist vorstellbar, dass erfasste Bilddaten und/oder Bilder des Kiefergelenks hierfür mit einem Signal des zumindest einen Sensors, der Information über die Bewegung des Kiefergelenks und/oder einem Zeitstempel korreliert bzw. assoziiert sind. Vorzugsweise umfassen auch das Signal des zumindest einen Sensors und/oder die Information über die Bewegung des Kiefergelenks einen Zeitstempel bzw. einen Zeitbezug, welcher ein Zuordnen der Bilddaten oder Bilder des Kiefergelenks zu einer Öffnungsstellung oder einer Mehrzahl von Öffnungsstellungen des Kiefergelenks ermöglicht.

Durch den Einsatz eines Motion-Gating-Verfahrens lässt sich ein Rechenaufwand für das Rekonstruieren der Bilder des Kiefergelenks auf vorteilhafte Weise reduzieren.

Das erfindungsgemäße Computerprogrammprodukt ist direkt in einen Speicher einer Recheneinheit einer erfindungsgemäßen Bildgebungsvorrichtung ladbar, mit Programmcode-Mitteln, um ein erfindungsgemäßes Verfahren auszuführen, wenn das Computerprogrammprodukt in der Recheneinheit der Bildgebungsvorrichtung ausgeführt wird.

Durch das erfindungsgemäße Computerprogrammprodukt kann das erfindungsgemäße Verfahren schnell, identisch wiederholbar, korrekt und robust ausgeführt werden. Das Computerprogrammprodukt ist so konfiguriert, dass es mittels der Recheneinheit die erfindungsgemäßen Verfahrensschritte ausführen kann. Die Recheneinheit muss dabei jeweils die Voraussetzungen, wie beispielsweise einen entsprechenden Arbeitsspeicher, eine entsprechende Grafikkarte oder eine entsprechende Logikeinheit aufweisen, so dass die jeweiligen Verfahrensschritte effizient ausgeführt werden können. Das Computerprogrammprodukt ist beispielsweise auf einem computerlesbaren Medium gespeichert oder auf einem Netzwerk, einem Server oder einer Cloud hinterlegt. Das Computerprogrammprodukt kann auf dem Server oder der Cloud ausführbar sein, aber auch zur Ausführung in einen Prozessor einer lokalen Recheneinheit geladen werden. Die Recheneinheit kann dabei als eine eigenständige Systemkomponente oder als ein Teil der Bildgebungsvorrichtung ausgebildet sein. Weiterhin können Steuerinformationen des Computerprogrammprodukts auf einem elektronisch lesbaren Datenträger gespeichert sein. Die Steuerinformationen des elektronisch lesbaren Datenträgers können derart ausgestaltet sein, dass sie bei Verwendung des Datenträgers in der Recheneinheit der Bildgebungsvorrichtung ein erfindungsgemäßes Verfahren durchführen. Beispiele für elektronisch lesbare Datenträger sind eine DVD, ein Magnetband, ein USB-Stick oder beliebige andere Datenspeicher, auf welchen elektronisch lesbare Steuerinformationen, insbesondere Software, gespeichert ist. Wenn diese Steuerinformationen von dem Datenträger gelesen und an eine Steuereinheit und/oder die Recheneinheit der Bildgebungsvorrichtung übertragen werden, können alle erfindungsgemäßen Ausführungsformen des beschriebenen, erfindungsgemäßen Verfahrens durchgeführt werden.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine schematische Repräsentation einer Ausführungsform einer erfindungsgemäßen Bildgebungsvorrichtung,
- Fig. 2: eine schematische Repräsentation einer Ausführungsform eines erfindungsgemäßen Beißelements,
- Fig. 3: eine schematische Repräsentation einer Ausführungsform eines erfindungsgemäßen Beißelements,
- Fig. 4: eine schematische Repräsentation einer Ausführungsform eines erfindungsgemäßen Beißelements,
- Fig. 5: eine schematische Repräsentation einer Ausführungsform eines erfindungsgemäßen Beißelements,
- Fig. 6: ein Ablaufschema einer Ausführungsform eines erfindungsgemäßen Verfahrens,
- Fig. 7: eine schematische Darstellung von Messwerten eines Sensors eines erfindungsgemäßen Beißelements,
- Fig. 8: eine schematische Repräsentation einer Ausführungsform eines erfindungsgemäßen Beißelements.

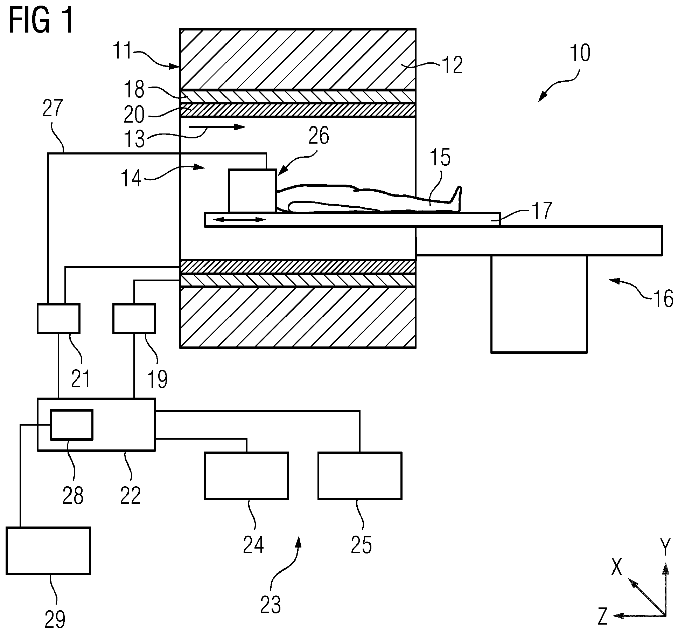

Fig. 1 zeigt eine mögliche Ausführungsform einer erfindungsgemäßen Bildgebungsvorrichtung 10. In dem gezeigten Beispiel ist die Bildgebungsvorrichtung 10 als eine Magnetresonanzvorrichtung 10 ausgestaltet. Es ist aber ebenso vorstellbar, dass die Bildgebungsvorrichtung 10 als eine Computertomographievorrichtung, eine Röntgenvorrichtung, eine Digitalkamera oder dergleichen ausgestaltet ist. Die Magnetresonanzvorrichtung 10 umfasst eine Magneteinheit 11, welche z. B. einen Permanentmagneten, einen Elektromagneten oder einen supraleitenden Hauptmagneten 12 zur Erzeugung eines starken und insbesondere homogenen Grundmagnetfelds 13 (B0-Magnetfeld) aufweist. Zudem umfasst die Magnetresonanzvorrichtung 10 einen Patientenaufnahmebereich 14 zu einer Aufnahme eines Untersuchungsobjekts 15, welches vorzugsweise ein Patient darstellt. Der Patientenaufnahmebereich 14 ist in dem vorliegenden Ausführungsbeispiel zylinderförmig ausgebildet und in einer Umfangsrichtung von der Magneteinheit 11 umgeben. Grundsätzlich sind jedoch auch von diesem Beispiel abweichende Ausbildungen des Patientenaufnahmebereichs 14 vorstellbar.

Der Patient 15 kann mittels einer Patientenlagerungsvorrichtung 16 der Magnetresonanzvorrichtung 10 in dem Patientenaufnahmebereich 14 positioniert werden. Die Patientenlagerungsvorrichtung 16 weist hierfür einen innerhalb des Patientenaufnahmebereichs 14 bewegbar ausgestalteten Patiententisch 17 auf. Die Magneteinheit 11 weist weiterhin eine Gradientenspule 18 zum Erzeugen von magnetischen Gradientenfeldern auf, welche für eine Ortskodierung von erfassten Magnetresonanzsignalen während einer Magnetresonanzuntersuchung verwendet wird. Die Gradientenspule 18 wird mittels einer Gradientensteuereinheit 19 der Magnetresonanzvorrichtung 10 angesteuert. Die Magneteinheit 11 kann weiterhin eine Hochfrequenzantenne umfassen, welche im vorliegenden Ausführungsbeispiel als fest in die Magnetresonanzvorrichtung 10 integrierte Körperspule 20 ausgebildet ist. Die Körperspule 20 ist zu einer Anregung von Atomkernen ausgelegt, die sich in dem von dem Hauptmagneten 12 erzeugten Grundmagnetfeld 13 befinden. Die Körperspule 20 wird von einer Hochfrequenzeinheit 21 der Magnetresonanzvorrichtung 10 angesteuert und strahlt hochfrequente Signale in eine Bildaufnahmeregion ein, welche im Wesentlichen von einem Patientenaufnahmebereich 14 der Magnetresonanzvorrichtung 10 gebildet ist. Die Körperspule 20 kann weiterhin auch zu einem Empfangen von Magnetresonanzsignalen ausgebildet sein.

Für eine Steuerung des Hauptmagneten 12, der Gradientensteuereinheit 19 und der Hochfrequenzeinheit 21 weist die Magnetresonanzvorrichtung 10 eine Steuereinheit 22 auf. Die Steuereinheit 22 ist dazu ausgebildet eine Durchführung einer Sequenz, wie z. B. einer bildgebenden GRE (gradient echo) Sequenz, einer TSE (turbo spin echo) Sequenz oder einer UTE (ultra short echo time) Sequenz, zu steuern. Zudem umfasst die Steuereinheit 22 eine Recheneinheit 28, welche dazu ausgebildet ist, digitalisierte Magnetresonanzsignale auszuwerten, welche während der Magnetresonanzuntersuchung erfasst werden.

Des Weiteren umfasst die Magnetresonanzvorrichtung 10 eine Benutzerschnittstelle 23, welche eine Signalverbindung mit der Steuereinheit 22 aufweist. Steuerinformationen, wie beispielsweise Bildgebungsparameter, aber auch rekonstruierte Bilder eines Kiefergelenks und/oder einer Kieferregion, können auf einer Ausgabeeinheit 24 der Benutzerschnittstelle 23 für einen Nutzer angezeigt werden. Die Ausgabeeinheit 24 kann hierfür zumindest einen Monitor und/oder Bildschirm umfassen. Weiterhin weist die Benutzerschnittstelle 23 eine Eingabeeinheit 25 auf, mittels der Parameter einer Magnetresonanzuntersuchung von dem Nutzer eingegeben werden können.

In dem gezeigten Beispiel weist die Magnetresonanzvorrichtung 10 eine als Kopfspule 26 ausgeführte Anordnung von Antennen auf, welche an dem Kopf des Patienten 15 positioniert ist. Die Kopfspule 26 ist dazu ausgebildet, Magnetresonanzsignale aus einem Volumen der Kopfregion des Patienten 15, insbesondere einem Volumen der Kieferregion und/oder des Kiefergelenks 43, zu erfassen und an die Magnetresonanzvorrichtung 10 zu übertragen. Die Kopfspule 26 weist vorzugsweise eine elektrische Anschlussleitung 27 auf, welche eine Signalverbindung mit der Hochfrequenzeinheit 21 und der Steuereinheit 22 bereitstellt. Ebenso wie die Körperspule 20 kann auch die Kopfspule 26 zu einem Anregen von Atomkernen und zu einem Empfangen von Magnetresonanzsignalen ausgebildet sein. Zum Aussenden von hochfrequenten Signalen wird eine Sendeeinheit der Kopfspule 26 von der Hochfrequenzeinheit 21 angesteuert. Die Kopfspule 26 kann den Kopf des Patienten 15 außenumfänglich entlang einer Längsachse des Patienten 15 umschließen. Es ist jedoch ebenso vorstellbar, dass die Magnetresonanzvorrichtung 10 eine alternativ ausgestaltete Lokalspule 26 aufweist, welche zu einem Erfassen von Magnetresonanzsignalen der Kieferregion bzw. des Kiefergelenks 43 des Patienten 15 ausgebildet sind.

Die dargestellte Magnetresonanzvorrichtung 10 kann selbstverständlich weitere Komponenten umfassen, welche Magnetresonanzvorrichtungen üblicherweise aufweisen. Es ist ebenso vorstellbar, dass die Magnetresonanzvorrichtung 10 statt des zylinderförmigen Aufbaus einen C-förmigen, einen dreieckigen oder einen asymmetrischen Aufbau der magnetfelderzeugenden Komponenten aufweist. Die Magnetresonanzvorrichtung 10 kann insbesondere eine dedizierte Magnetresonanzvorrichtung 10 sein, welche dazu ausgebildet ist, eine Magnetresonanzuntersuchung der Kieferregion und/oder des Kiefergelenks 43 eines stehenden oder sitzenden Patienten 15 durchzuführen.

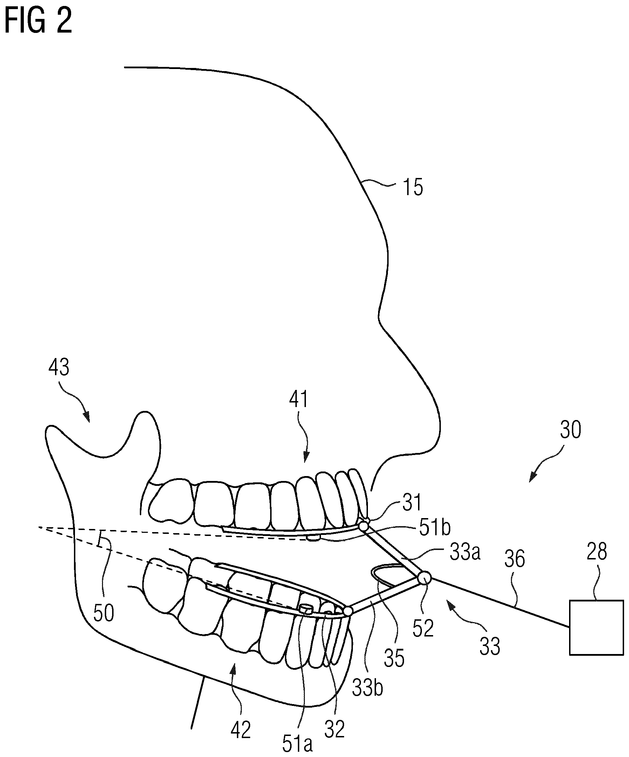

Fig. 2 zeigt eine schematische Repräsentation eines erfindungsgemäßen Beißelements 30. Das Beißelement 30 weist vorliegend eine Kopplungseinheit 33 mit einem Gelenk 52 auf. Die Kopplungseinheit 33 ist mittels zwei Armen bzw. Trägern 33a und 33b mechanisch mit einem ersten Abschnitt 31 und einem zweiten Abschnitt 32 verbunden. Zwischen den beiden Armen bzw. Trägern 33a und 33b ist ein Rückstellglied 35 vorgesehen, welches vorliegend als eine Feder ausgeführt ist. Das Rückstellglied 35 ist dazu ausgebildet, einer Schließbewegung des Kiefergelenks 43 des Patienten 15 einen vorbestimmten Widerstand entgegenzusetzen. Das Rückstellglied 35 kann ferner dazu ausgebildet sein, den ersten Abschnitt 31 sowie den zweiten Abschnitt 32 dem Oberkiefer 41 und/oder dem Unterkiefer 42 bei einer Öffnungsbewegung des Kiefergelenks 43 nachzuführen. Das Beißelement 30 kann somit in der anwendungsgemäßen Position an der Kieferregion des Patienten 15 gehalten werden. Mittels des Rückstellglieds 35 können der erste Abschnitt 31 und der zweite Abschnitt 32 ferner kraftschlüssig mit dem Oberkiefer 41 und dem Unterkiefer 42 verbunden werden.

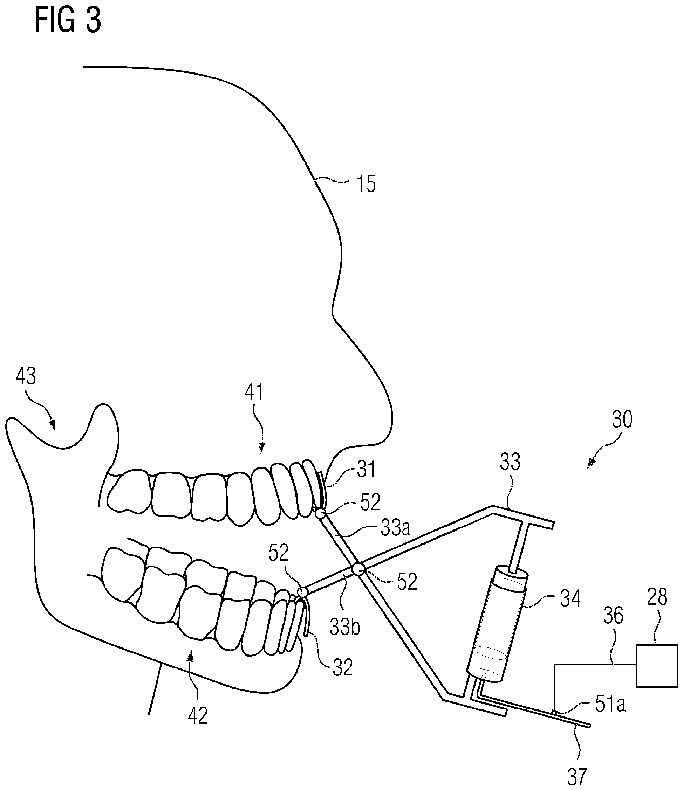

Der erste Abschnitt 31 und der zweite Abschnitt 32 können mittels weiterer Gelenke 52 (siehe Fig. 3) mit der Kopplungseinheit 33 verbunden sein. Im gezeigten Beispiel sind der erste Abschnitt 31 und der zweite Abschnitt 32 als U-förmige Plättchen ausgeführt. Die U-förmigen Plättchen können aus einem rigiden Material bestehen und eine adhäsive Teilfläche aufweisen, welche eine stoffschlüssige Verbindung des ersten Abschnitts 31 und des zweiten Abschnitts 32 mit den Zahnbögen des Oberkiefers 41 und das Unterkiefers 42 ermöglicht.

In der gezeigten Ausführungsform ist das Gelenk 52 der Kopplungseinheit 33 bei anwendungsgemäßer Positionierung des Beißelements 30 außerhalb einer Mundhöhle des Patienten 15 positioniert. In einer alternativen Ausführungsform kann das Gelenk 52 des Beißelements 30 aber auch in der Mundhöhle des Patienten 15 positioniert sein.

Das Beißelement 30 weist vorliegend zwei Sensoren 51a und 51b auf. Die Sensoren 51a und 51b sind vorzugsweise als Abstandssensoren ausgeführt, welche dazu ausgebildet sind, den Abstand zwischen dem ersten Abschnitt 31 und dem zweiten Abschnitt 32 in Abhängigkeit eines reflektierten Lichtsignals und/oder eines reflektierten Ultraschallsignals zu ermitteln.

Ein mittels der Sensoren 51a und/oder 51b erfasster Messwert und/oder eine in Abhängigkeit des erfassten Messwerts ermittelte Information über die Bewegung des Kiefergelenks 43 kann über die Signalverbindung 36 an die Recheneinheit 28 der Bildgebungsvorrichtung 10 übermittelt werden. Es ist vorstellbar, dass die Recheneinheit 28 dazu ausgebildet ist, die Öffnungsstellung des Kiefergelenks 43, welche durch den Winkel 50 zwischen dem Oberkiefer 41 und dem Unterkiefer 42 charakterisiert ist, anhand von Messwerten der Sensoren 51a und 51b zu ermitteln. Weiterhin können auch die Sensoren 51a und 51b bereits eine Recheneinheit aufweisen, welche dazu ausgebildet ist, den Winkel 50 bzw. die Information über die Bewegung des Kiefergelenks 43 zu ermitteln.

Fig. 3 zeigt eine Ausführungsform des erfindungsgemäßen Beißelements 30, bei welcher die Kopplungseinheit 33 ein Dämpfungselement 34 aufweist. Das Dämpfungselement 34 ist vorliegend als ein Hohlzylinder ausgeführt, in welchem ein Kolben verschiebbar gelagert ist. Der Kolben ist derart mechanisch mit der Kopplungseinheit 33 verbunden, dass eine Schließbewegung des Kiefergelenks 43 eine Verschiebung des Kolbens innerhalb des Hohlzylinders verursacht, wodurch ein Fluid in dem Hohlzylinder bzw. der Fluidleitung 37 komprimiert und/oder verdrängt wird. Die Fluidleitung 37 weist dabei einen Drucksensor 51a auf, welcher dazu ausgebildet ist, einen Fluiddruck in der Fluidleitung 37 zu ermitteln. Der Drucksensor 51a kann den ermittelten Fluiddruck mittels der Signalverbindung 36 an die Recheneinheit 28 übermitteln. Die Recheneinheit 28 kann entsprechend dazu ausgebildet sein, die Information über die Bewegung des Kiefergelenks 43 in Abhängigkeit des Fluiddrucks zu bestimmen.

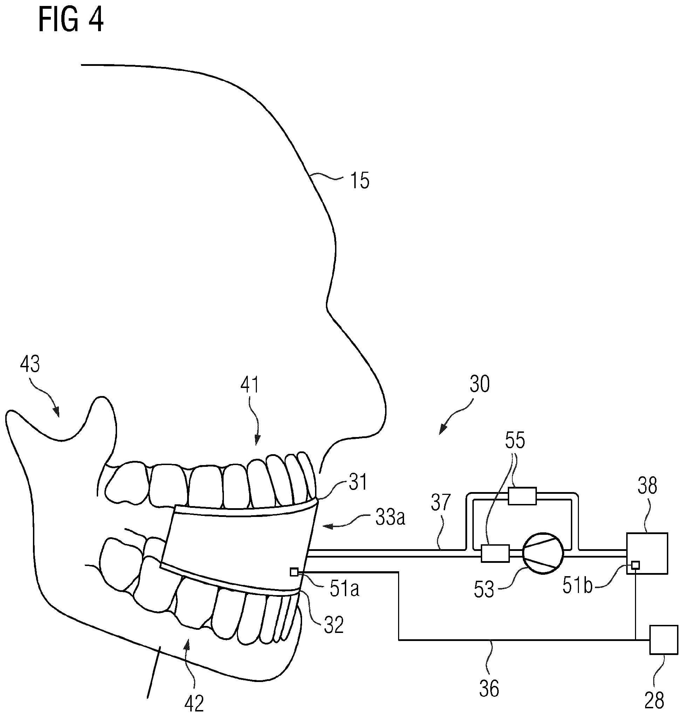

In einer Ausführungsform umfasst das Dämpfungselement 34 ein kompressibles Fluid, welches in dem Hohlzylinder des Dämpfungselements eingeschlossen ist. Eine Fluidleitung 37 gemäß Fig. 3 kann dabei fehlen, sodass das kompressible Fluid der Schließbewegung des Kiefergelenks 43 einen vorbestimmten Widerstand entgegensetzt. In einer bevorzugten Ausführungsform sind der Hohlzylinder und die Fluidleitung 37 dagegen mit einem inkompressiblen Fluid befüllt, welches bei der Schließbewegung des Kiefergelenks 43 aus dem Hohlzylinder in die Fluidleitung 37 verdrängt wird. Die Fluidleitung 37 kann dabei ein elastisches oder druckbehaftetes Fluidreservoir und/oder ein Förderaggregat 53 aufweisen (siehe Fig. 4), welche dazu ausgebildet sind, das Fluid bei einer Öffnungsbewegung des Kiefergelenks 43 zurück in den Hohlzylinder zu fördern und/oder einen vorbestimmten Widerstand bereitzustellen. Es ist vorstellbar, dass die Fluidleitung 37 geeignete Armaturen, wie z. B. Ventile und/oder Rückschlagklappen 55 (siehe Fig. 4), aufweist, um einen Fluss des Fluids in der Fluidleitung 37 in Abhängigkeit einer Öffnungsbewegung und/oder einer Schließbewegung des Kiefergelenks 43 anzupassen.

Der erste Abschnitt 31 und der zweite Abschnitt 32 sind vorliegend als U-förmige Klammern ausgestaltet, welche jeweils einen oder mehrere Zähne des Oberkiefers 41 und des Unterkiefers 42 an einer Zahninnenseite, einer Kaufläche und einer Zahnaußenseite umgreifen. Die U-förmigen Klammern können insbesondere dazu ausgebildet sein, eine kraftschlüssige und/oder eine formschlüssige Verbindung mit dem entsprechenden Zahn bzw. den entsprechenden Zähnen bereitzustellen. Weiterhin sind der erste Abschnitt 31 sowie der zweite Abschnitt 32 mittels Gelenken 52a und 52b mit den Armen bzw. Trägern 33a und 33b verbunden, um eine Änderung der geometrischen Konfiguration der Kopplungseinheit 33 während der Bewegung des Kiefergelenks zu kompensieren.

In der in Fig. 4 gezeigten Ausführungsform weist das erfindungsgemäße Beißelement 30 ein mit einem Fluid befülltes Kissen 33a auf, welches in der Mundhöhle des Patienten 15 positioniert ist. Das Fluid in dem Kissen 33a kann mittels der Fluidleitung 37 über ein Förderaggregat 53 mit einem Fluidreservoir 38 verbunden sein. Das Förderaggregat 53 kann insbesondere dazu ausgebildet sein, einen Fluiddruck in dem Kissen 33a auf einen vorbestimmten Wert einzustellen. Der Wert kann dabei konstant sein oder sich in Abhängig der Bewegung des Kiefergelenks 43 und/oder einer Randbedingung der Bildgebungsuntersuchung ändern.

Die Fluidleitung 37 kann, wie oben beschrieben, weitere Armaturen aufweisen, um einen Fluidfluss in Abhängigkeit der Bewegung des Kiefergelenks anzupassen und/oder zu optimieren. Beispielsweise kann die Fluidleitung 37 einen Bypass aufweisen, welcher mittels einer Anordnung von Rückschlagklappen 55 während einer Schließbewegung des Kiefergelenks 43 eine Fluidströmung aus dem Kissen 33a an dem Förderaggregat 53 vorbei in das Fluidreservoir 38 ermöglicht. Gleichermaßen kann die Anordnung von Rückschlagklappen 55 eine rückwärtige Durchströmung des Förderaggregats 53 bei einer Schließbewegung des Kiefergelenks 43 verhindern.

Das Fluidreservoir 38 weist vorliegend einen Füllstandsensor 51b auf, welcher den Füllstand in dem Fluidreservoir 38 erfasst. Zusätzlich kann das Kissen 33a einen Drucksensor 51a aufweisen, welcher den Fluiddruck in dem Kissen 33a erfasst. Die Messwerte der beiden Sensoren 51a und 51b werden mittels der Signalverbindung 36 an die Recheneinheit 28 übertragen. Die Recheneinheit 28 ist dazu ausgebildet, die Information über die Bewegung des Kiefergelenks 43 in Abhängigkeit des Füllstands in dem Fluidreservoir 38 und/oder des Fluiddrucks in dem Kissen 33a zu ermitteln.

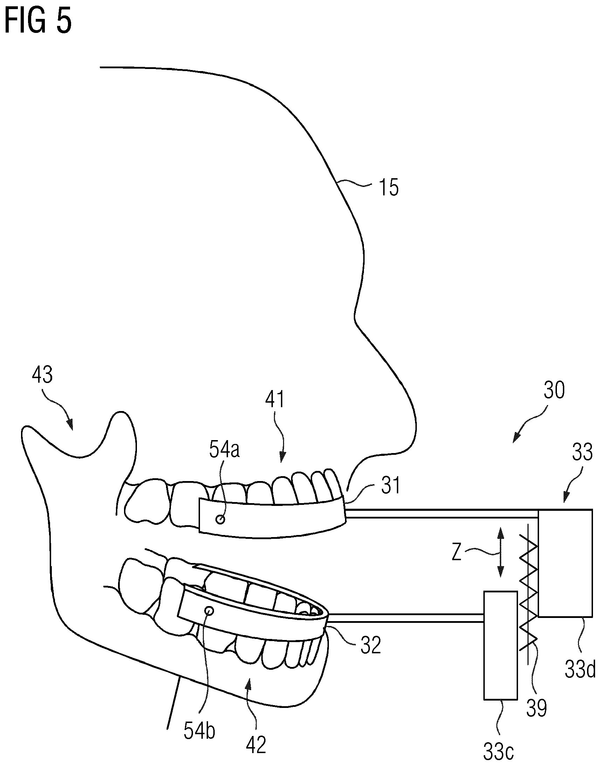

Das Kissen 33a ist vorzugsweise aus einem elastischen und/oder flexiblen Material gefertigt, welches sich bei einer Schließbewegung des Kiefergelenks 43 zusammenfalten bzw. zusammenschieben lässt. In einer Ausführungsform stellen der erste Abschnitt 31 und/oder der zweite Abschnitt 32 lediglich in Richtung der Zahnbögen des Patienten 15 ausgerichtete Seiten des Kissens 33a dar. Es ist aber ebenso vorstellbar, dass der erste Abschnitt 31 und/oder der zweite Abschnitt 32 eine im Wesentlichen planare Gestalt und ein rigides Material aufweisen, welche dem Kissen 33a zumindest an den in Richtung der Zahnbögen ausgerichteten Seiten eine strukturelle Stabilität verleihen. Es ist weiterhin vorstellbar, dass der erste Abschnitt 31 und/oder der zweite Abschnitt 32 einem Verlauf und/oder einem Profil des Zahnbogen des Patienten 15 nachgeformt sind und/oder diesen zumindest teilweise umgreifen (siehe Fig. 5). Das Kissen 33a weist vorzugsweise eine Aussparung (nicht gezeigt) auf, um eine Behinderung einer Zunge des Patienten 15 zu vermeiden.

Fig. 5 zeigt eine Ausführungsform des erfindungsgemäßen Beißelements 30, bei welcher der erste Abschnitt 31 und der zweite Abschnitt 32 den Zahnbogen des Unterkiefers 42 und den Zahnbogen des Oberkiefers 41 zumindest teilweise entlang einer Kaufläche und einer Zahnaußenseite umgreifen. Vorzugsweise weisen in Richtung der anliegenden Zahnbögen ausgerichtete Teilflächen des ersten Abschnitts 31 und/oder des zweiten Abschnitts 32 eine plastische Masse auf (nicht gezeigt). Die plastische Masse kann dazu ausgebildet sein, bei anwendungsgemäßer Positionierung des Beißelements 30 an der Kieferregion verformt zu werden und eine formschlüssige und/oder kraftschlüssige Verbindung mit dem jeweiligen Zahnbogen bereitzustellen.

Die Kopplungseinheit 30 weist in dem gezeigten Beispiel einen Linearantrieb 39 auf, welcher dazu ausgebildet ist, den ersten Abschnitt 31 relativ zu dem zweiten Abschnitt 32 zu positionieren. Ein Anpassen der relativen Position des ersten Abschnitts 31 zu dem zweiten Abschnitt 32 kann insbesondere ein Öffnen des Kiefergelenks 43 des Patienten 15 mittels des Linearantriebs 39b umfassen. Es ist vorstellbar, dass die Kopplungseinheit 33 einen Mechanismus aufweist, welcher einen Bypass des Linearantriebs 39b oder einen Freilauf des ersten Abschnitts 31 zu dem zweiten Abschnitt 32 ermöglicht, z. B. wenn eine maximale Auslenkung des Linearantriebs 39 erreicht und/oder wenn ein zulässiger Gegendruck auf den Oberkiefer 41 und/oder den Unterkiefer 42 überschritten wurde. Der Linearantrieb 39 ist vorzugsweise dazu ausgestaltet, das Element 33a der Kopplungseinheit 33 zumindest entlang der Z-Richtung relativ gegenüber dem Element 33c zu positionieren, wobei der erste Abschnitt 31 relativ zu dem zweiten Abschnitt 32 positioniert wird.

Der erste Abschnitt 31 ist in den gezeigten Ausführungsformen mit dem Zahnbogen des Oberkiefers 41 verbunden. Es ist aber ebenso vorstellbar, dass der erste Abschnitt 31 mit einer Stirnregion, einer Wangenregion, einer Kinnregion und/oder einer beliebigen anderen Region des Kopfes des Patienten 15 verbunden und/oder einer solchen nachgeformt ist. Der erste Abschnitt 31 kann insbesondere dazu ausgebildet sein, den Kopf des Patienten 15 während der Durchführung einer Bildgebungsuntersuchung zu fixieren, sodass lediglich der Unterkiefer 42 des Patienten 15 beweglich ist.

Der erste Abschnitt 31 und der zweite Abschnitt 32 weisen ferner Marker 54a und 54b auf, welche mittels einer Magnetresonanzuntersuchung abbildbar sind. Die Marker 54a und 54b können aktive Marker, wie z. B. eine Spule oder eine Antenne, umfassen. Eine Spule oder Antenne kann dazu ausgebildet sein, ein hochfrequentes Signal in einem Frequenz- und Leistungsbereich einer Magnetresonanzuntersuchung zu emittieren. Die Marker 54a und 54b können aber auch passive Marker, wie z. B. Ölkapseln oder Kapseln mit Vitaminlösungen, umfassen. Solche passiven Marker emittieren ein hochfrequentes Signal erst nach Anregung durch ein vorbestimmtes hochfrequentes Signal, welche beispielsweise von der Körperspule 20 der Magnetresonanzvorrichtung 10 bereitgestellt wird. Die Recheneinheit 28 kann dazu ausgebildet sein, die Öffnungsstellung des Kiefergelenks 43 und/oder den Winkel 50 in Abhängigkeit einer relativen Position des Markers 54a zu dem Marker 54b zu ermitteln. Die relative Position des Markers 54a zu dem Marker 54b kann dabei anhand erfasster Bilddaten und/oder Bilder des Kiefergelenks 43 bestimmt werden. Die Marker 54 können wie in Fig. 5 mit dem ersten Abschnitt 31 und/oder dem zweiten Abschnitt 32 verbunden sein. Es ist aber ebenso vorstellbar, dass zumindest ein Marker 54 mit der Kopplungseinheit 33, insbesondere einem Arm bzw. Träger 33a und/oder 33b (siehe Fig. 2, Fig. 3), verbunden ist.

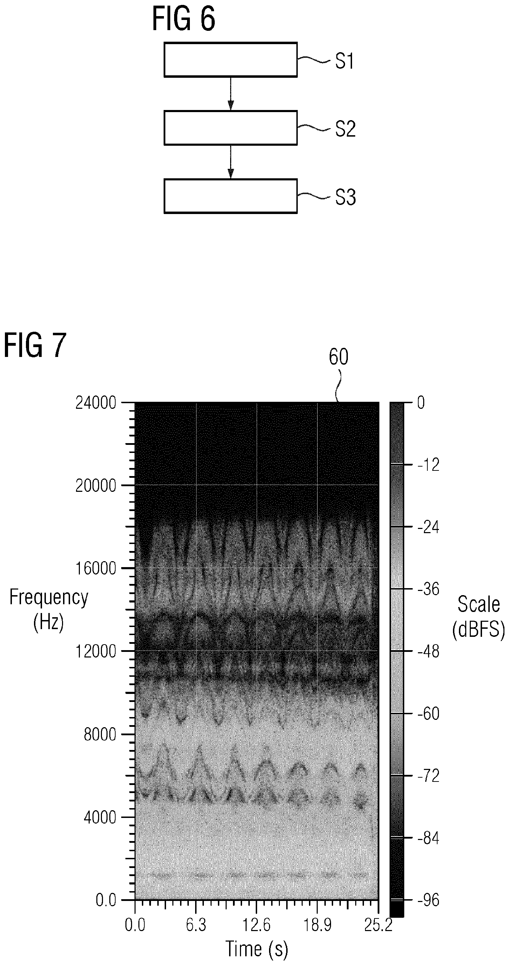

Fig. 6 zeigt ein Ablaufdiagramm einer Ausführungsform eines erfindungsgemäßen Verfahrens zur Durchführung einer dynamischen Bildgebungsuntersuchung eines Kiefergelenks 43 eines Patienten 15 während einer Bewegung des Kiefergelenks 15 mit einer Bildgebungsvorrichtung 10 und einem Beißelement 30, wobei das Beißelement 30 in einer anwendungsgemäßen Position an der Kieferregion des Patienten 15 positioniert ist.

Ein vorbereitender Schritt des Verfahrens umfasst vorzugsweise ein Positionieren des Beißelements 30 in der anwendungsgemäßen Position an der Kieferregion des Patienten 15 sowie ein Positionieren des Patienten 15 in der Bildaufnahmeregion der Bildgebungsvorrichtung 10 mittels der Patientenlagerungsvorrichtung 16.

In dem Schritt S1 des erfindungsgemäßen Verfahrens erfolgt ein Erfassen von Bilddaten des Kiefergelenks 43 des Untersuchungsobjekts 15 mittels der Bildgebungsvorrichtung 10 während eines Variierens der relativen Position des ersten Abschnitts 31 zu dem zweiten Abschnitt 32 des Beißelements 30. Zu dem Erfassen der Bilddaten des Kiefergelenks 43 können beliebige Bildgebungssequenzen eingesetzt werden, welche für eine Aufnahme von Bilddaten des Kiefergelenks 43 geeignet sind. Das Erfassen der Bilddaten des Kiefergelenks erfolgt vorzugsweise während einer im Wesentlichen kontinuierlichen oder diskontinuierlichen Bewegung des Kiefergelenks 15 des Patienten 15. Eine diskontinuierliche Bewegung des Kiefergelenks 43 kann dabei durch ein unstetiges, aber regelmäßig ablaufendes oder sich wiederholendes, Variieren einer relativen Position des Oberkiefers 41 zu dem Unterkiefer 42 charakterisiert sein. Die Bewegung des Kiefergelenks 43 des Patienten 15 kann autark oder manuell durch den Patienten 15 erfolgen. Es ist aber ebenso vorstellbar, dass die Bewegung des Kiefergelenks 43 des Patienten 15 zumindest teilweise von einem Antrieb 39, einem Dämpfungselement 34 und/oder einem Rückstellglied 35 des Beißelements 30 unterstützt werden.

In einer Ausführungsform umfasst das Erfassen von Bilddaten des Kiefergelenks 43 einen Einsatz eines Motion-Gating-Verfahrens. Dies kann insbesondere bedeuten, dass das Erfassen der Bilddaten des Kiefergelenks 43 in Abhängigkeit eines vorbestimmten Werts und/oder Wertebereichs eines Sensors 51 getriggert bzw. initiiert wird. Dadurch lässt sich das Erfassen der Bilddaten des Kiefergelenks auf vorbestimmte Öffnungsstellungen des Kiefergelenks 43 beschränken.

In dem Schritt S2 erfolgt ein Erfassen der Information über die Bewegung des Kiefergelenks 43 mittels des zumindest einen Sensors 51. Das Erfassen der Information über die Bewegung des Kiefergelenks 43 kann beispielsweise ein Erfassen des Abstands zwischen dem ersten Abschnitt 31 und dem zweiten Abschnitt 32, einer Kraftaufnahme des Kopplungselements 33, des Rückstellglieds 35, des Dämpfungselements 34 und/oder eines Fluiddrucks umfassen. Es ist ebenso vorstellbar, dass eine Recheneinheit 28 der Bildgebungsvorrichtung 10 oder eine Recheneinheit des Sensors 51 dazu ausgebildet ist, die Information über die Bewegung des Kiefergelenks 43 in Abhängigkeit des Messwerts des zumindest einen Sensors 51 zu ermitteln. Die Information über die Bewegung des Kiefergelenks 43 kann beispielsweise einen Winkel 50 zwischen dem Unterkiefer 42 und dem Oberkiefer 41 und/oder eine prozentuale Quantifizierung der Öffnungsstellung des Kiefergelenks 43 umfassen. Vorzugsweise umfasst die Information über die Bewegung des Kiefergelenks ferner eine Zeitinformation bzw. einen Zeitstempel, welcher mit der Bewegung des Kiefergelenks 43 sowie mit den erfassten Messwerten korreliert ist.

In einer Ausführungsform umfasst das Erfassen der Information über die Bewegung des Kiefergelenks 43 ein Erfassen von Bilddaten des zumindest einen Markers 54. Es ist vorstellbar, dass anhand der Bilddaten des zumindest einen Markers 54 Bilder rekonstruiert werden. Die Bilder können mittels eines Bildverarbeitungsalgorithmus der Recheneinheit 28 verarbeitet werden, um eine relative Position des zumindest einen Markers 54 zu einer anatomischen Struktur und/oder einem weiteren Marker 54 zu ermitteln. Vorzugsweise bestimmt die Recheneinheit 28 in Abhängigkeit der relativen Position des zumindest einen Markers 54 zu der anatomischen Struktur und/oder dem weiteren Marker 54 die Öffnungsstellung des Kiefergelenks 43.

Bevorzugt überlagern sich die Schritte S1 und S2 des erfindungsgemäßen Verfahrens zeitlich zumindest teilweise. Bei dem Erfassen der Bilddaten des Kiefergelenks 43 können Magnetresonanzsignale oder k-Raum Daten kontinuierlich aufgenommen werden (z. B. mehrfach dieselbe k-Raumzeile), während zeitsynchron oder parallel dazu ein Signal des zumindest einen Sensors 51, welches mit einer Öffnungsstellung des Kiefergelenks 43 korreliert ist, aufgenommen wird.

Es ist vorstellbar, dass sich eine vorbestimmte Zahl an zu rekonstruierenden Bildern des Kiefergelenks 43 ergibt, indem das Signal des zumindest einen Sensors 51 gemäß einer oben beschriebenen Ausführungsform entsprechenden Öffnungsstellungen des Kiefergelenks 43 zugeordnet wird. Aufgenommene Magnetresonanzsignale können bei dem Erfassen der Bilddaten des Kiefergelenks 43 mittels eines Motion-Gating-Verfahrens entsprechend in die jeweiligen k-Räume in Abhängigkeit des Signals des zumindest einen Sensors 51 aufgeteilt werden. Dies kann bedeuten, dass gemessene k-Raum Zeilen bereits beim Erfassen der Bilddaten des Kiefergelenks 43 einzelnen Öffnungsstellungen des Kiefergelenks 43 zugeordnet werden. Das Erfassen der Bilddaten des Kiefergelenks 43 mittels des Motion-Gating-Verfahrens kann somit ein Erfassen von k-Raum Daten für unterschiedliche Öffnungsstellungen des Kiefergelenks 43 umfassen.

In dem Schritt S3 des erfindungsgemäßen Verfahrens erfolgt ein Rekonstruieren von Bildern des Kiefergelenks 43 des Patienten 15 in Abhängigkeit der Information über die Bewegung des Kiefergelenks 43. Hierbei können anhand des Motion-Gating-Verfahrens erfasste und den Öffnungsstellungen des Kiefergelenks 43 zugeordnete k-Raum Daten für die Rekonstruktion der vorbestimmten Zahl an zu rekonstruierenden Bildern des Kiefergelenks 43 herangezogen werden. Weiterhin kann das Rekonstruieren von Bildern des Kiefergelenks 43 in Abhängigkeit der Information über die Bewegung des Kiefergelenks 43 ein Zuordnen von Bildern und/oder Bilddaten des Kiefergelenks 43 zu vorbestimmten Öffnungsstellungen, insbesondere verschiedenen Winkeln 50, des Kiefergelenks 43 umfassen. Die einer Öffnungsstellung des Kiefergelenks 43 zugeordneten Bildern oder Bilddaten können auch überlagert und/oder zu einer Quantifizierung bzw. Korrektur von Bildartefakten sowie zu einer gegenseitigen Ergänzung der Bilddaten oder Bilder herangezogen werden.

In einer Ausführungsform umfasst das Rekonstruieren der Bilder des Kiefergelenks 43 ein Ermitteln einer Mehrzahl von Öffnungsstellungen des Kiefergelenks 43 in Abhängigkeit der Information über die Bewegung des Kiefergelenks 43, wobei die Bilder des Kiefergelenks 43 der Mehrzahl von Öffnungsstellungen des Kiefergelenks 43 zugeordnet werden.

Das Zuordnen der Bilder und/oder Bilddaten zu der Mehrzahl von Öffnungsstellungen des Kiefergelenks erfolgt vorzugsweise in Abhängigkeit von vorbestimmten Werten und/oder Wertebereichen von Signalen des Sensors 51 bzw. daraus abgeleiteter Informationen über die Bewegung des Kiefergelenks 43. Vorzugsweise wird in Abhängigkeit von Messwerten des zumindest einen Sensors 51 eine zeitabhängige Bewegung und/oder eine Bewegungshistorie des Kiefergelenks 43 ermittelt. Dabei werden einzelne Bilder des Kiefergelenks 43 in Abhängigkeit eines Zeitstempels der Mehrzahl von Öffnungsstellungen des Kiefergelenks 43 zugeordnet. Es ist vorstellbar, dass hierbei Verfahren der Interpolation oder der Ausgleichsrechnung eingesetzt werden. Das Zuordnen der Bilder des Kiefergelenks 43 kann weiterhin auch in Abhängigkeit eines Modells des Unterkiefers und/oder Oberkiefers, aber auch in Abhängigkeit einer Position eines Markers 54 erfolgen.

In einer weiteren Ausführungsform kann auch das Rekonstruieren von Bildern des Kiefergelenks 43 einen Einsatz eines Motion-Gating-Verfahrens umfassen. Vorzugsweise erfolgt ein Auswählen von erfassten Bilddaten des Kiefergelenks 43, welche für das Rekonstruieren von Bildern des Kiefergelenks 43 dedizierter Öffnungsstellungen des Kiefergelenks 43 verwendet werden, hierbei in Abhängigkeit von vorbestimmten Messwerten und/oder Messwertbereichen des Sensors 51 und/oder der Information über die Bewegung des Kiefergelenks 43.

Fig. 7 zeigt schematisch einen Einfluss einer Bewegung des Kiefergelenks 43 auf ein akustisches Signal oder ein Referenzsignal, welches in der Mundhöhle des Patienten 15 ausgesendet wurde. Das Frequenzspektrum ("Frequency") und der Signalpegel ("Scale") des akustischen Signals wurden mittels eines Schallwandlers eines akustischen Sensors 51 in der Mundhöhle des Patienten 15 erfasst und in Abhängigkeit der Zeit ("Time") aufgetragen. Wie in dem Schaubild 60 zu erkennen ist, verändert sich der Signalpegel des empfangenen akustischen Signals oder Referenzsignals bei einem wiederholenden Öffnen und Schließen des Kiefergelenks 43 aufgrund einer Veränderung der Resonanzeigenschaften und/oder akustischen Eigenschaften der Mundhöhle. Anhand der in dem Schaubild 60 gezeigten Abhängigkeit lässt sich die Information über die Bewegung des Kiefergelenks 43 ermitteln. Es ist vorstellbar, dass hierfür, z. B. im Vorfeld der Bildgebungsuntersuchung, eine Kalibrierung des akustischen Sensors 51 in der Mundhöhle des Patienten 15 durchgeführt wird. Es ist weiterhin vorstellbar, dass aus den zeitabhängigen Messwerten eine Bewegungshistorie des Kiefergelenks 43 bestimmt wird, anhand derer die Öffnungsstellung des Kiefergelenks 43 für beliebige Zeitpunkte ermittelt werden kann. Dies kann insbesondere mittels einer Interpolation oder Ausgleichsrechnung erfolgen. Ein akustischer Sensor 51 kann, wie in Fig. 1 gezeigt, zumindest mit dem zweiten Abschnitt 32 verbunden oder frei in der Mundhöhle des Patienten 15 positioniert sein.

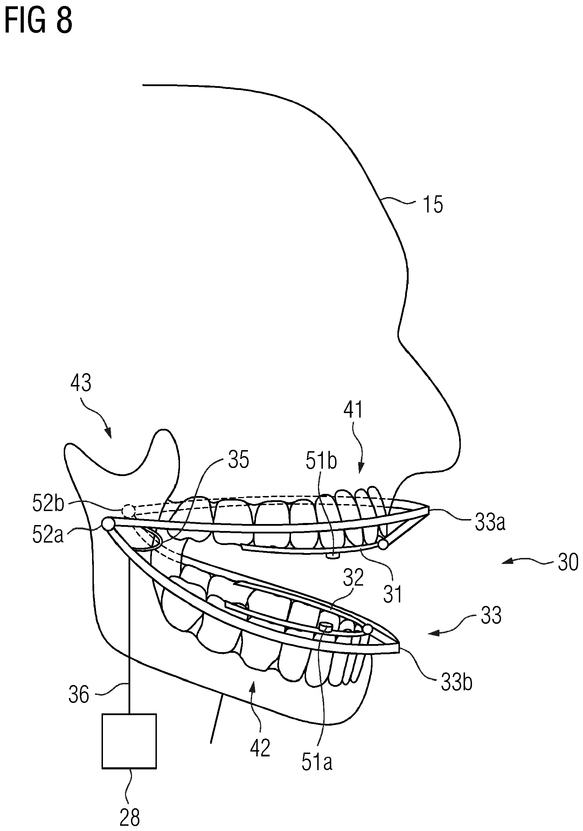

Fig. 8 zeigt schematisch eine Ausführungsform, bei welchem das Gelenk 52 der Kopplungseinheit 33 bei anwendungsgemäßer Positionierung des Beißelements 30 an einer Wangenregion des Patienten 15 positioniert ist. Das Gelenk 52 ist dabei außerhalb der Mundhöhle des Patienten an dem Kiefergelenk 43 und/oder einer Wangenregion des Patienten angeordnet. In diesem Fall kann die Kopplungseinheit 33 eine oder mehrere Spangen bzw. Bügel 33a, 33b aufweisen, welche den ersten Abschnitt 31 und den zweiten Abschnitt 32 mechanisch mit dem Gelenk 52 verbinden. Bevorzugt weist das Beißelement 30 bei dieser Ausführungsform ein erstes Gelenk 52a und ein zweites Gelenk 52b auf. Bei anwendungsgemäßer Positionierung des Beißelements 30 ist das zweite Gelenk 52b an einer linken Seite der Kieferregion des Patienten 15 positioniert, während das erste Gelenk 52a an einer rechten Seite der Kieferregion des Patienten 15 positioniert ist. Der erste Abschnitt 31 kann dabei mittels eines ersten Bügels 33a mechanisch mit dem ersten Gelenk 52a und dem zweiten Gelenk 52b verbunden sein. Entsprechend kann auch der zweite Abschnitt 32 mittels eines zweiten Bügels 33b mit dem ersten Gelenk 52a und dem zweiten Gelenk 52b verbunden sein. Durch Öffnen und Schließen des Kiefergelenks 43 werden der erste Abschnitt 31 und der zweite Abschnitt 32, aber auch der erste Bügel 33a und der zweite Bügel 33b, jeweils unter Öffnen und Schließen der Gelenke 52a und 52b, relativ zueinander bewegt. Ein Quantifizieren dieser relativen Bewegung kann mittels eines Sensors 51 gemäß einer hierin beschriebenen Ausführungsform erfolgen.

Selbstverständlich sind die hier beschriebenen Ausführungsformen des erfindungsgemäßen Verfahrens und der erfindungsgemäßen Magnetresonanzvorrichtung als beispielhaft zu verstehen. Einzelne Ausführungsformen sind daher um Merkmale anderer Ausführungsformen erweiterbar. Insbesondere ist die Reihenfolge der Verfahrensschritte des erfindungsgemäßen Verfahrens als beispielhaft zu verstehen. Die einzelnen Schritte können auch in einer anderen Reihenfolge durchgeführt werden oder sich zeitlich teilweise oder vollständig überschneiden.

## Patentansprüche

1. Beißelement (30) zur Unterstützung einer dynamischen Bildgebungsuntersuchung eines Kiefergelenks (43) eines Untersuchungsobjekts, umfassend einen ersten Abschnitt (31), einen zweiten Abschnitt (32), eine Kopplungseinheit (33) und zumindest einen Sensor (51), wobei bei anwendungsgemäßer Positionierung des Beißelements (30) an einer Kieferregion des Untersuchungsobjekts der erste Abschnitt (31) in einer vorbestimmten relativen Position zu einem Oberkiefer (41) des Untersuchungsobjekts positioniert ist und der zweite Abschnitt (32) mechanisch mit einem Unterkiefer (42) des Untersuchungsobjekts verbunden ist, wobei der erste Abschnitt (31) und der zweite Abschnitt (32) mechanisch mittels der Kopplungseinheit (33) miteinander verbunden sind und in Abhängigkeit einer Bewegung des Kiefergelenks (43) relativ zueinander positionierbar sind und wobei der zumindest eine Sensor (51) dazu ausgebildet ist, eine Information über die Bewegung des Kiefergelenks (43) in Abhängigkeit der relativen Position des ersten Abschnitts (31) zu dem zweiten Abschnitts (32) zu ermitteln,
**dadurch gekennzeichnet, dass**
die Kopplungseinheit (33) dazu ausgebildet ist, der Bewegung des Kiefergelenks (43) unter Veränderung einer geometrischen Konfiguration einen vorbestimmten Widerstand entgegenzusetzen, wobei der zumindest eine Sensor (51) dazu ausgebildet ist, die Information über die Bewegung des Kiefergelenks (43) in Abhängigkeit der geometrischen Konfiguration der Kopplungseinheit (33) zu ermitteln.

2. Beißelement (30) nach Anspruch 1, wobei die Kopplungseinheit (33) ein Dämpfungselement (34) aufweist, wobei die Kopplungseinheit (33) dazu ausgebildet ist, eine mit einer Relativbewegung des ersten Abschnitts (31) zu dem zweiten Abschnitt (32) verbundene Kraft auf das Dämpfungselement (34) zu übertragen, wobei das Dämpfungselement (34) dazu ausgebildet ist, den vorbestimmten Widerstand bereitzustellen und eine Geschwindigkeit der Bewegung des Kiefergelenks (43) zu begrenzen.

3. Beißelement (30) nach einem der vorhergehenden Ansprüche, wobei die Kopplungseinheit (33) ein mit einem Fluid gefülltes Kissen aufweist, wobei bei anwendungsgemäßer Positionierung des Beißelements (30) der erste Abschnitt (31) auf einer dem Oberkiefer (41) zugewandten Seite des Kissens und der zweite Abschnitt (32) auf einer dem Unterkiefer (42) zugewandten Seite des Kissens angeordnet sind, wobei der vorbestimmte Widerstand von einem Fluiddruck des Fluids in dem Kissen abhängt und wobei das Fluid in dem Kissen mittels einer Fluidleitung (37) mit einem Förderaggregat (53) gekoppelt ist, welches dazu ausgebildet ist, den Fluiddruck in dem Kissen konstant oder dynamisch einzustellen.

4. Beißelement (30) nach einem der vorhergehenden Ansprüche, wobei die Kopplungseinheit (33) einen Antrieb (39) aufweist, welcher dazu ausgebildet ist, den vorbestimmten Widerstand der Kopplungseinheit (33), die geometrische Konfiguration der Kopplungseinheit (33) und/oder die relative Position des ersten Abschnitts (31) zu dem zweiten Abschnitt (32) einzustellen.

5. Beißelement (30) nach Anspruch 4, wobei der Antrieb (39) als ein elektrischer Antrieb, ein hydraulischer Antrieb, ein pneumatischer Antrieb oder ein Linearantrieb ausgestaltet ist.

6. Beißelement (30) nach einem der vorhergehenden Ansprüche, wobei der zumindest eine Sensor (51) einen Kraftaufnehmer, einen Positionsgeber, einen optischen Sensor, einen Füllstandsensor, einen akustischen Sensor, einen Ultraschallsensor und/oder einen Hall-Sensor aufweist.

7. Beißelement (30) nach einem der vorhergehenden Ansprüche, ferner aufweisend zumindest einen Marker (54), welcher dazu ausgebildet ist, mittels der Bildgebungsuntersuchung abgebildet zu werden, wobei der zumindest eine Marker (54) derart mechanisch mit dem Beißelement (30) verbunden ist, dass der zumindest eine Marker (54) bei der Bewegung des Kiefergelenks (43) relativ zu dem ersten Abschnitt (31) positionierbar ist.

8. Bildgebungsvorrichtung (10) mit einem Beißelement (30) nach einem der vorhergehenden Ansprüche, wobei die Bildgebungsvorrichtung (10) dazu ausgebildet ist, eine dynamische Bildgebungsuntersuchung eines Kiefergelenks (43) eines Untersuchungsobjekts während einer Bewegung des Kiefergelenks (43) durchzuführen.

9. Verfahren zur Durchführung einer dynamischen Bildgebungsuntersuchung eines Kiefergelenks (43) eines Untersuchungsobjekts mit einer Bildgebungsvorrichtung (10) nach Anspruch 8 und einem Beißelement (30) nach einem der Ansprüche 1 bis 7, wobei das Beißelement (30) in einer anwendungsgemäßen Position an der Kieferregion des Untersuchungsobjekts positioniert ist, umfassend die folgenden Schritte:
• Erfassen (S1) von Bilddaten des Kiefergelenks (43) des Untersuchungsobjekts bei verschiedenen relativen Positionen des ersten Abschnitts (31) zu dem zweiten Abschnitt (32) des Beißelements (30) mittels der Bildgebungsvorrichtung,
• Erfassen (S2) der Information über die Bewegung des Kiefergelenks (43) mittels des zumindest einen Sensors (51) und
• Rekonstruieren (S3) von Bildern des Kiefergelenks (43) des Untersuchungsobjekts in Abhängigkeit der Information über die Bewegung des Kiefergelenks (43).

10. Verfahren nach Anspruch 9, wobei das Rekonstruieren (S3) der Bilder des Kiefergelenks (43) ein Ermitteln einer Mehrzahl von Öffnungsstellungen des Kiefergelenks (43) in Abhängigkeit der Information über die Bewegung des Kiefergelenks (43) umfasst und wobei die Bilder des Kiefergelenks (43) der Mehrzahl von Öffnungsstellungen des Kiefergelenks (43) zugeordnet werden.

11. Verfahren nach Anspruch 10 mit einem Beißelement nach Anspruch 7, wobei das Erfassen (S2) der Information über die Bewegung des Kiefergelenks (43) ein Erfassen von Bilddaten des zumindest einen Markers (54) umfasst und wobei das Rekonstruieren (S3) der Bilder des Kiefergelenks (43) ein Ermitteln der Mehrzahl von Öffnungsstellungen des Kiefergelenks (43) in Abhängigkeit der erfassten Bilddaten des zumindest einen Markers (54) umfasst.

12. Verfahren nach Anspruch 9, wobei das Erfassen (S1) von Bilddaten des Kiefergelenks (43) und/oder das Rekonstruieren (S3) von Bildern des Kiefergelenks (43) einen Einsatz eines Motion-Gating-Verfahrens umfassen.

13. Computerprogrammprodukt, welches direkt in einen Speicher einer Recheneinheit einer Bildgebungsvorrichtung (10) nach Anspruch 8 ladbar ist, mit Programmcode-Mitteln, um ein Verfahren nach einem der Ansprüche 9 bis 12 auszuführen, wenn das Computerprogrammprodukt in der Recheneinheit der Bildgebungsvorrichtung (10) ausgeführt wird.

## Claims

1. Biting element (30) for supporting a dynamic imaging examination of a temporomandibular joint (43) of an examination object, comprising a first section (31), a second section (32), a coupling unit (33) and at least one sensor (51), wherein when the biting element (30) is positioned as intended on a jaw region of the examination object, the first section (31) is positioned in a predetermined position relative to an upper jaw (41) of the examination object and the second section (32) is connected mechanically to a lower jaw (42) of the examination object, wherein the first section (31) and the second section (32) are connected mechanically to one another by means of the coupling unit (33) and can be positioned relative to one another as a function of a movement of the temporomandibular joint (43), and wherein the at least one sensor (51) is embodied to determine an item of information relating to the movement of the temporomandibular joint (43) as a function of the position of the first section (31) relative to the second section (32),
**characterised in that**
the coupling unit (33) is embodied to oppose the movement of the temporomandibular joint (43) by changing a geometric configuration a predetermined resistance, wherein the at least one sensor (51) is embodied to determine the information about the movement of the temporomandibular joint (43) as a function of the geometric configuration of the coupling unit (33).

2. Biting element (30) according to claim 1, wherein the coupling unit (33) has a damping element (34), wherein the coupling unit (33) is embodied to transmit a force connected to a movement of the first section (31) relative to the second section (32) to the damping element (34), wherein the damping element (34) is embodied to provide the predetermined resistance and to limit a speed of the movement of the temporomandibular joint (43).

3. Biting element (30) according to one of the preceding claims, wherein the coupling unit (33) has a cushion filled with a fluid, wherein when the biting element (30) is positioned as intended, the first section (31) is arranged on a side of the cushion facing the upper jaw (41) and the second section (32) is arranged on a side of the cushion facing the lower jaw (42), wherein the predetermined resistance depends on a fluid pressure of the fluid in the cushion, and wherein the fluid in the cushion is coupled to a delivery unit (53) by means of a fluid line (37), said delivery unit (53) being embodied to set the fluid pressure in the cushion constantly or dynamically.

4. Biting element (30) according to one of the preceding claims, wherein the coupling unit (33) has a drive (39), which is embodied to set the predetermined resistance of the coupling unit (33), the geometric configuration of the coupling unit (33) and/or the position of the first section (31) relative to the second section (32).

5. Biting element (30) according to claim 4, wherein the drive (39) is designed as an electrical drive, a hydraulic drive, a pneumatic drive or a linear drive.

6. Biting element (30) according to one of the preceding claims, wherein the at least one sensor (51) has a force transducer, a position sensor, an optical sensor, a fill level sensor, an acoustic sensor, an ultrasound sensor and/or a Hall sensor.

7. Biting element (30) according to one of the preceding claims, further having at least one marker (54), which is embodied to be mapped by means of the imaging examination, wherein the at least one marker (54) is mechanically connected to the biting element (30) such that when the temporomandibular joint (43) moves the at least one marker (54) can be positioned relative to the first section (31).

8. Imaging apparatus (10) with a biting element (30) according to one of the preceding claims, wherein the imaging apparatus (10) is embodied to carry out a dynamic imaging examination of a temporomandibular joint (43) of an examination object during a movement of the temporomandibular joint (43).

9. Method for carrying out a dynamic imaging examination of a temporomandibular joint (43) of an examination object with an imaging apparatus (10) according to claim 8 and a biting element (30) according to one of claims 1 to 7, wherein the biting element (30) is positioned in an intended position on the jaw region of the examination object, comprising the following steps:
• Detecting (S1) image data of the temporomandibular joint (43) of the examination object with different positions of the first section (31) relative to the second section (32) of the biting element (30) by means of the imaging apparatus,
• Detecting (S2) the information about the movement of the temporomandibular joint (43) by means of the at least one sensor (51), and
• Reconstructing (S3) images of the temporomandibular joint (43) of the examination object as a function of the information about the movement of the temporomandibular joint (43) .

10. Method according to claim 9, wherein the reconstruction (S3) of the images of the temporomandibular joint (43) comprises a determination of a plurality of opening positions of the temporomandibular joint (43) as a function of the information about the movement of the temporomandibular joint (43) and wherein the images of the temporomandibular joint (43) are assigned to the plurality of opening positions of the temporomandibular joint (43).

11. Method according to claim 10 with a biting element according to claim 7, wherein the detection (S2) of the information about the movement of the temporomandibular joint (43) comprises a detection of image data of the at least one marker (54) and wherein the reconstruction (S3) of the images of the temporomandibular joint (43) comprises a determination of the plurality of opening positions of the temporomandibular joint (43) as a function of the detected image data of the at least one marker (54).

12. Method according to claim 9, wherein the detection (S1) of image data of the temporomandibular joint (43) and/or the reconstruction (S3) of images of the temporomandibular joint (43) comprise a use of a motion gating method.

13. Computer program product, which can be loaded directly into a memory of a computing unit of an imaging apparatus (10) according to claim 8, having program code means in order to execute a method according to one of claims 9 to 12, when the computer program product is executed in the computing unit of the imaging apparatus (10).

## Revendications

1. Elément (30) à mordre pour venir à l'appui d'un examen d'imagerie dynamique d'une articulation (43) temporo-mandibulaire d'un objet à examiner, comprenant une première partie (31), une deuxième partie (32), une unité (33) de liaison et au moins un capteur (51), dans lequel, en position conforme à l'utilisation de l'élément (30) à mordre sur une région de mâchoire de l'objet à examiner, la première partie (31) est positionnée en une position relative déterminée à l'avance par rapport à un maxillaire (41) supérieur de l'objet à examiner et la deuxième partie (32) est reliée mécaniquement à un maxillaire (42) inférieur de l'objet à examiner, dans lequel la première partie (31) et la deuxième partie (32) sont reliées l'une à l'autre mécaniquement au moyen de l'unité (33) de liaison et peuvent être mises en position l'une par rapport à l'autre en fonction d'un déplacement de l'articulation (43) temporo-mandibulaire et dans lequel le au moins un capteur (51) est constitué pour déterminer une information sur le déplacement de l'articulation (43) temporo-mandibulaire en fonction de la position relative de la première partie (31) par rapport à la deuxième partie (32),
**caractérisé en ce que**
l'unité (33) de liaison est constituée pour opposer une résistance déterminée à l'avance au déplacement de l'articulation (43) temporo-mandibulaire, par modification d'une configuration géométrique, dans lequel le au moins un capteur (51) est constitué pour déterminer de l'information sur le mouvement de l'articulation (43) temporo-mandibulaire en fonction de la configuration géométrique de l'unité (33) de liaison.

2. Elément (30) à mordre suivant la revendication 1, dans lequel l'unité (33) de liaison a un élément (34) d'amortissement, dans lequel l'unité (33) de liaison est constituée pour transmettre à l'élément (34) d'amortissement une force reliée au mouvement relatif de la première partie (31) par rapport à la deuxième partie (32), dans lequel l'élément (34) d'amortissement est constitué pour disposer d'une résistance déterminée à l'avance et pour limiter une vitesse du déplacement de l'articulation (43) temporo-mandibulaire.

3. Elément (30) à mordre suivant l'une des revendications précédentes, dans lequel l'unité (33) de liaison a un coussin rempli d'un fluide, dans lequel, dans la mise en position conforme à l'utilisation de l'élément (30) à mordre, la première partie (31) est disposée d'un côté, tourné vers le maxillaire (41) supérieur, du coussin et la deuxième partie (32) d'un côté, tourné vers le maxillaire (42) inférieur, du coussin, dans lequel la résistance déterminée à l'avance dépend d'une pression du fluide dans le coussin et dans lequel le fluide dans le coussin est, au moyen d'un conduit (37) pour du fluide, relié à un groupe (53) de refoulement, qui est constitué pour régler dynamiquement ou à une valeur constante la pression du fluide dans le coussin.

4. Elément (30) à mordre suivant l'une des revendications précédentes, dans lequel l'unité (33) de liaison a un entraînement (39), qui est constitué pour régler la résistance déterminée à l'avance de l'unité (33) de liaison, la configuration géométrique de l'unité (33) de liaison et/ou la position relative de la première partie (31) par rapport à la deuxième partie (32).

5. Elément (30) à mordre suivant la revendication 4, dans lequel l'entraînement (39) est sous la forme d'un entraînement électrique, d'un entraînement hydraulique, d'un entraînement pneumatique ou d'un entraînement linéaire.

6. Elément (30) à mordre suivant l'une des revendications précédentes, dans lequel le au moins un capteur (51) a un enregistreur de force, un transmetteur de position, un capteur optique, un capteur de niveau, un capteur acoustique, un capteur par ultrasons et/ou un capteur de Hall.

7. Elément (30) à mordre suivant l'une des revendications précédentes, comportant en outre au moins un marqueur (54), qui est conformé pour être représenté au moyen de l'examen d'imagerie, dans lequel le au moins un marqueur (54) est relié mécaniquement à l'élément (30) à mordre, de manière à pouvoir mettre le au moins un marqueur (54) en position par rapport à la première partie (31), lors du déplacement de l'articulation (43) temporo-mandibulaire.

8. Installation (10) d'imagerie comprenant un élément (30) à mordre suivant l'une des revendications précédentes, dans lequel l'installation (10) d'imagerie est constituée pour effectuer un examen d'imagerie dynamique d'une articulation (43) temporo-mandibulaire d'un objet à examiner pendant un déplacement de l'articulation (43) temporo-mandibulaire.

9. Procédé pour effectuer un examen d'imagerie dynamique d'une articulation (43) temporo-mandibulaire d'un objet à examiner par une installation (10) d'imagerie suivant la revendication 8 et par un élément (30) à mordre suivant l'une des revendications 1 à 7, dans lequel l'élément (30) à mordre est, dans une position conforme à l'utilisation, en position sur la région de mâchoire de l'objet à examiner, comprenant les stades suivants :
• saisie (S1) de données d'image de l'articulation (43) temporo-mandibulaire de l'objet à examiner en des positions relatives différentes de la première partie (31) par rapport à la deuxième partie (32) de l'élément (30) à mordre, au moyen de l'installation d'imagerie,
• saisie (S2) de l'information sur le déplacement de l'articulation (43) temporo-mandibulaire, au moyen du au moins un capteur (51) et
• reconstruction (S3) d'images de l'articulation (43) temporo-mandibulaire de l'objet à examiner en fonction de l'information sur le déplacement de l'articulation (43) temporo-mandibulaire.

10. Procédé suivant la revendication 9, dans lequel la reconstruction (S3) des images de l'articulation (43) temporo-mandibulaire comprend une détermination d'une pluralité de positions d'ouverture de l'articulation (43) temporo-mandibulaire en fonction de l'information sur le déplacement de l'articulation (43) temporo-mandibulaire et dans lequel les images de l'articulation (43) temporo-mandibulaire sont affectées à la pluralité de positions d'ouverture de l'articulation (43) temporo-mandibulaire.

11. Procédé suivant la revendication 10 comprenant un élément à mordre suivant la revendication 7, dans lequel la saisie (S2) de l'information sur le déplacement de l'articulation (43) temporo-mandibulaire comprend une saisie de données d'image du au moins un marqueur (54) et dans lequel la reconstruction (S3) des images de l'articulation (43) temporo-mandibulaire comprend une détermination de la pluralité de positions d'ouverture de l'articulation (43) temporo-mandibulaire en fonction des données d'image saisies du au moins un marqueur (54).

12. Procédé suivant la revendication 9, dans lequel la saisie (S1) de données d'image de l'articulation (43) temporo-mandibulaire et/ou la reconstruction (S3) d'images de l'articulation (43) temporo-mandibulaire comprend la mise en œuvre d'un procédé motion-gating.

13. Produit de programme d'ordinateur, qui peut être chargé directement dans la mémoire d'une unité informatique d'une installation (10) d'imagerie suivant la revendication 8, comprenant des moyens de code de programme pour exécuter un procédé suivant l'une des revendications 9 à 12, lorsque le produit de programme d'ordinateur est exécuté dans l'unité informatique de l'installation (10) d'imagerie.
